(19) Europäisches Patentamt
European Patent Office
Office européen des brevets

(11) EP 4 693 436 A1

(12) **EUROPEAN PATENT APPLICATION**
published in accordance with Art. 153(4) EPC

(43) Date of publication:
**11.02.2026 Bulletin 2026/07**

(21) Application number: **24856758.8**

(22) Date of filing: **16.08.2024**

(51) International Patent Classification (IPC):
***H01M 4/13*** *(2010.01)* ***H01M 4/62*** *(2006.01)*
***H01M 10/42*** *(2006.01)* ***H01M 10/052*** *(2010.01)*
***H01M 50/586*** *(2021.01)* ***H01M 4/02*** *(2006.01)*

(52) Cooperative Patent Classification (CPC):
**H01M 4/02; H01M 4/13; H01M 4/62; H01M 10/052; H01M 10/42; H01M 50/586;** Y02E 60/10

(86) International application number:
**PCT/KR2024/012240**

(87) International publication number:
**WO 2025/042146 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.08.2023 KR 20230108455**

(71) Applicants:
- **LG Chem, Ltd.**
  **Seoul 07336 (KR)**
- **LG Energy Solution, Ltd.**
  **Seoul 07335 (KR)**

(72) Inventors:
- **KANG, Joon Koo**
  **Daejeon 34122 (KR)**
- **SONG, In Taek**
  **Daejeon 34122 (KR)**
- **KOH, Jong Kwan**
  **Daejeon 34122 (KR)**
- **YANG, Hee Myeong**
  **Daejeon 34122 (KR)**
- **KIM, Ki Hwan**
  **Daejeon 34122 (KR)**
- **LEE, Yu Mi**
  **Daejeon 34122 (KR)**
- **CHO, Woo Hyung**
  **Daejeon 34122 (KR)**
- **LEE, Seok Kyeong**
  **Daejeon 34122 (KR)**

(74) Representative: **Plasseraud IP**
**104 Rue de Richelieu**
**CS92104**
**75080 Paris Cedex 02 (FR)**

(54) **ELECTRODE**

(57) The present specification discloses an electrode and a use thereof. The electrode comprises a polymer layer exhibiting a so-called PTC (Positive Temperature Coefficient) effect at a required level at a required time, whereby it can be suitably applied in applications where stability problems due to abnormally high heat or flame are caused. The polymer layer can exhibit oxidation potential characteristics suitable for an electrode use, where such oxidation potential characteristics can be stably maintained even under harsh environments. In the polymer layer of the electrode, the PTC effect can rapidly appear at a required time. The present specification also discloses a use of the electrode.

[Figure 1]

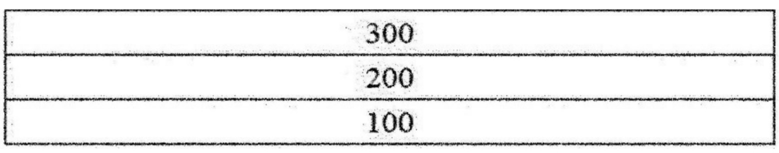


EP 4 693 436 A1

## Description

### Technical Field

This application claims the benefit of priority based on Korean Patent Application No. 10-2023-0108455 dated August 18, 2023, the disclosure of which is incorporated herein by reference in its entirety.
The present specification discloses an electrode and a use thereof.

### Background Art

An energy storage technology has expanded application areas to mobile phones, tablet and notebook PCs, or electric vehicles, and the like.
As the data processing speed of mobile devices such as mobile phones or tablets increases and their used hours also increase, the development of secondary batteries with high energy density and operating potential, long cycle life, and low self-discharge rate is in progress.
As major developed countries suppress production of cars driven by internal combustion engines to alleviate global warming and air pollution, major automobile manufacturers also develop various electric vehicles, and thus the importance of secondary batteries having high energy density, high discharge voltage, and output stability increases as driving sources thereof.
According to the above trend, the occurrence frequency of ignition or explosion accidents due to overcharging, high-temperature exposure or outer shocks, and the like in devices or automobiles using secondary batteries as an energy source also increases.
As a main cause of such accidents, a short phenomenon, in which a positive electrode and a negative electrode inside an electrode assembly come into direct contact with each other mainly due to external stimuli, is known. When the secondary battery is overcharged or exposed to high temperatures or external stimuli, the short phenomenon can occur by the separator shrinkage due to the internal temperature increase of the secondary battery, or the internal structure destruction of the secondary battery due to outer shocks, and the like.
When the short phenomenon occurs, migration of lithium ions and electrons is concentrated through the region where the positive electrode and the negative electrode are in direct contact with each other, so that internal heat generation can be promoted. Accordingly, it is known that as gas or the like is generated inside the battery, the volume expands, and the risk of ignition increases.

### Disclosure

### Technical Problem

The present specification discloses an electrode and a use thereof. It is an object of the present specification to disclose an electrode applied with a polymer layer capable of securing stability in applications where stability problems due to abnormally high heat or flame are caused by exhibiting a so-called PTC (Positive Temperature Coefficient) effect at a required level at a required time. It is another object of the present specification to disclose an electrode comprising a polymer layer which exhibits oxidation potential characteristics suitable for applications and stably maintains such oxidation potential characteristics even under harsh environments. It is another object of the present specification to disclose an electrode comprising a polymer layer in which the PTC effect rapidly appears at a desired level at a required time. It is another object of the present specification to disclose a use of the electrode.

### Technical Solution

In this specification, the term room temperature means a natural temperature without artificially warming or cooling. The room temperature may be, for example, any temperature within a range of 10°C to 30°C, or a temperature of about 23°C, about 25°C, or about 27°C or so.
Among physical properties mentioned in this specification, the physical property affected by the measurement temperature is a physical property measured at room temperature, unless otherwise specified.
The unit of temperature in this specification is Celsius (°C), unless otherwise specified.
In this specification, the term normal pressure means a natural pressure without artificially pressurizing or depressurizing, where a pressure within a range of about 730 mmHg to 790 mmHg or so may be generally regarded as the normal pressure.
Among physical properties mentioned in this specification, the physical property affected by the measurement pressure is a physical property measured at normal pressure, unless otherwise specified.

In this specification, the standard-state humidity means any relative humidity within a range of 40% to 60%, where for example, a relative humidity of about 40%, 45%, 50%, 55%, or 60% may be referred to as the standard-state humidity.

Among physical properties mentioned in this specification, the physical property affected by the measurement humidity is a physical property measured at the standard-state humidity, unless otherwise specified.

In this specification, the term normal state means a normal operating state (e.g., a normal charging or discharging state of a secondary battery) and/or a storage state of an electrical/electronic device such as a secondary battery.

In this specification, the term abnormal state means a state where abnormal heating, ignition, and/or explosion occurs in an electrical/electronic device such as secondary batteries, or the risk of the abnormal heating, ignition, and/or explosion is increased. For example, a state where abnormal heating, ignition, or explosion occurs due to a short circuit phenomenon in a secondary battery, or a dangerous state where the possibility of the heating, ignition, or explosion is increased may be the abnormal state.

The present specification discloses an electrode.

The electrode may comprise a current collector and an electrode active material layer formed on the current collector. The electrode may further comprise a polymer layer between the current collector and the electrode active material layer. Figure 1 is a cross-sectional schematic diagram of an electrode comprising a current collector (100), a polymer layer (200), and an electrode active material layer (300).

In the electrode, the current collector (100) and the polymer layer (200), and the polymer layer (200) and the active material layer (300) may be in contact with each other. In some cases, other elements may exist between the current collector (100) and the polymer layer (200) or between the polymer layer (200) and the active material layer (300). In addition, although the drawing shows a case in which the active material layer (300) exists only on one side of the current collector (100), the active material layer (300) may also exist on both sides of the current collector (100). In this case, the polymer layer (200) may also exist in two layers between each of the active material layers (300) present on both sides of the current collector (100) and the current collector (100), and may also exist in one layer between any one of the active material layers (300) present on both sides and the current collector (100).

The electrode may be, for example, a positive electrode (anode) or a negative electrode (cathode) applied to a secondary battery.

The polymer layer exhibits the so-called PTC (positive temperature coefficient) effect. Therefore, the polymer layer may variably control the movement of charges through the electrode depending on the temperature.

By applying such a polymer layer, the electrode is applied to a secondary battery, and the like, whereby it can exhibit excellent electrical characteristics including low resistance in a normal state, and secure stability through an increase in resistance in an abnormal state.

In order that the polymer layer is applied to the electrode, thereby exhibiting the effect, the oxidation potential of the polymer layer and the tendency of the PTC effect exhibited by the polymer layer must be controlled. The PTC effect is an effect in which resistance increases in proportion to temperature. The temperature at the time when the resistance increases due to the PTC effect and the resistance of the polymer layer before the resistance increase affect the performance of the secondary battery. For example, if the PTC effect is excessively expressed at the temperature in the normal state, the performance of the secondary battery cannot be properly expressed before stability is secured. In addition, if the oxidation potential of the polymer layer is not appropriately controlled in relation to the electrode active material, there is a problem that the performance of the battery, and the like in the normal state deteriorates.

The polymer layer exhibits a PTC effect, and the oxidation potential of the polymer layer having this PTC effect and the PTC effect do not affect the performance of the secondary battery in the normal state, and are controlled so that stability can be secured in the abnormal state.

As described below, the conductive polymer contains a unique functional group such as a relatively long-chain hydrocarbon functional group, a no-thiophene monomer unit, and the like, together with a thiophene monomer unit. By controlling the drying or annealing temperature in a process of forming the polymer layer comprising such a conductive polymer and the conductive material, an appropriate PTC effect (for example, inducing an increase in the resistance of the polymer layer at a desired temperature (the temperature of the abnormal state in the battery)) can be secured.

In one example, the polymer layer applied to the electrode may have a lower oxidation potential than the electrode active material layer (or the electrode active material included in the electrode active material layer). The oxidation potential mentioned in this specification is, unless otherwise specified, an oxidation potential based on $Li/Li^+$, which is measured in the manner described in the Example sections of this specification. The oxidation potential varies depending on the electrode and electrolyte applied for measurement, where the oxidation potential in this specification is an oxidation potential measured based on lithium and lithium ions ($Li/Li^+$).

The oxidation potential mentioned in this specification is the oxidation potential based on $Li/Li^+$, which is measured in the manner described in "4. Measurement of oxidation potential (polymer layer)" or "5. Measurement of oxidation potential (electrode active material)" of the Example sections of this specification.

In one example, in the electrode, the oxidation potential of the polymer layer may be controlled so that $\Delta V$ according to Equation 1 below is within a predetermined range.

[Equation 1]

$$\Delta V = 100 \times (Va - Vs) / Va$$

In Equation 1, Va is the oxidation potential of the active material layer or the electrode active material included therein, and Vs is the oxidation potential of the polymer layer.

The lower limit of ΔV in Equation 1 may be 0%, 0.5%, 1%, 1.5%, 2%, 2.5%, 3%, 3.5%, 4%, 4.5%, 5%, 5.5%, or 6% or so, and the upper limit thereof may be %, 20%, 19%, 18%, 17%, 16%, 15%, 14%, 13%, 12%, 11%, 10%, 9%, 8%, 7%, 6%, 5%, or 4.5% or so. The ΔV may be within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits. By controlling the oxidation potential of the polymer layer to satisfy the relationship, it is possible to provide a battery operating stably and efficiently in a normal state.

The oxidation potential of the polymer layer is not particularly limited if the above relationship is satisfied. The lower limit of the oxidation potential (Vs) of the polymer layer may be 2V, 2.1V, 2.2V, 2.3V, 2.4V, 2.5V, 2.6V, 2.7V, 2.8V, 2.9V, 3V, 3.1V, 3.2V, 3.3V, 3.4V, 3.5V, 3.6V, or 3.7V or so, and the upper limit thereof may also be 6V, 5.5V, 5V, 4.9V, 4.8V, 4.7V, 4.6V, 4.5V, 4.4V, 4.3V, 4.2V, 4.1V, 4.0V, 3.9V, 3.8V, 3.7V, 3.6V, or 3.5V or so. The oxidation potential may be within a range of less than or equal to, or less than any upper limit arbitrarily selected from the above-listed upper limits; or within a range of more than or equal to, or more than any lower limit arbitrarily selected from the above-listed lower limits while being less than or equal to, or less than any upper limit arbitrarily selected from the above-listed upper limits.

In one example, the polymer layer or the electrode to which the polymer layer is applied may have a DC resistance at 25°C in a certain level or less. Accordingly, stable operation or storage of the secondary battery in a normal state may be possible. The upper limit of the DC resistance may be 10,000, 9500, 9000, 8500, 8000, 7500, 7000, 6500, 6000, 5500, 5000, 4500, 4000, 3500, 3000, 2500, 2000, 1500, 1000, 950, 900, 850, 800, 750, 700, 650, 600, 550, 500, 450, 400, 350, 300, 250, 200, 150, 100, 90, 80, 70, or 60 or so, and the lower limit thereof may also be 5, 10, 50, 100, 150, 200, 250, 300, 350, 400, 450, 500, 550, or 600 or so. The DC resistance may be within a range of less than or equal to, or less than any upper limit arbitrarily selected from the above-listed upper limits; or within a range of more than or equal to, or more than any lower limit arbitrarily selected from the above-listed lower limits while being less than or equal to, or less than any upper limit arbitrarily selected from the above-listed upper limits. The unit of the DC resistance is Ω·cm, which is measured in the manner described in "6. DC resistance measurement method" of Example sections of this specification.

In one example, the polymer layer or the electrode of the polymer layer electrode may have an AC impedance resistance at 25°C in a certain level or less. Accordingly, stable operation or storage of the secondary battery may be possible in a normal state. The upper limit of the AC impedance resistance may be 1,000, 950, 900, 850, 800, 750, 700, 650, 600, 550, 500, 450, 400, 350, 300, 250, 200, 150, 100, 95, 90, 85, 80, 75, 70, 65, 60, 55, 50, 45, 40, 35, 30, 25, 20, 15, 10, 9, 8, or 7 or so, and the lower limit thereof may also be 1, 2, 3, 4, 5, 6, 7, 8, 9, or 10 or so. The AC impedance resistance may be within a range of less than or equal to, or less than any upper limit arbitrarily selected from the above-listed upper limits; or within a range of more than or equal to, or more than any lower limit arbitrarily selected from the above-listed lower limits while being less than or equal to, or less than any upper limit arbitrarily selected from the above-listed upper limits. The unit of the AC resistance is Ω, which is measured in the manner described in "7. AC impedance resistance" of Example sections of this specification.

By exhibiting the DC resistance and/or AC impedance resistance, the secondary battery to which the electrode is applied can be stably operated and stored in a normal state.

The electrode is applied to the battery, thereby exhibiting increased resistance in an abnormal state, and by blocking energization of the electrode assembly therethrough, it is possible to ensure stability.

The polymer layer or the electrode to which the polymer layer is applied may exhibit a characteristic in which ΔR1 of Equation 2 below is in a certain level or more.

[Equation 2]

$$\Delta R1 = Max\{(R_{n+5}/R_n)/5\}$$

In Equation 2, $R_n$ is the DC resistance at an arbitrary temperature n°C within a range of 25°C to 135°C, $R_{n+5}$ is the DC resistance at a temperature ((n+5)°C) that is 5°C higher than the temperature n°C, and Max{($R_{n+5}/R_n$)/5} is the maximum value among the ($R_{n+5}/R_n$)/5 values confirmed within the temperature range of 25°C to 135°C.

The method of measuring ΔR1 of Equation 2 is described in the "8. Measurement of maximum resistance change rate (DC resistance)" section of Examples sections. In the method of confirming ΔR1 above, the initial temperature is 25°C and the final temperature is 135°C. The $R_{n+5}$ and $R_n$ are confirmed by measuring the DC resistance at each temperature while increasing the temperature by 5°C from the initial temperature of 25°C. For example, when n is 90, $R_{95}/R_{90}$ is the ratio

of the DC resistance at 95°C to the DC resistance at 90°C. The matter that ΔR1 exhibits a certain level or more at any temperature within the temperature range of 25°C to 135°C means that the resistance increases relatively rapidly at any temperature within the temperature range.

The lower limit of ΔR1 above may be 10, 50, 100, 110, 120, 130, 140, 150, 160, 170, 180, or 190, and the upper limit thereof may also be 1,000, 950, 900, 850, 800, 750, 700, 650, 600, 550, 500, 450, 400, 350, 300, 250, 200, 190, or 180 or so. The unit of the ΔR1 is Ω·cm/°C. The ΔR1 may be within a range of more than or equal to, or more than any lower limit arbitrarily selected from the above-listed lower limits; or within a range of less than or equal to, or less than any upper limit arbitrarily selected from the above-listed upper limits while being more than or equal to, or more than any lower limit arbitrarily selected from the above-listed lower limits.

The temperature at which ΔR1 of the range is confirmed, that is, the temperature at $R_n$ of Equation 2, may be the range. The temperature range is very important in terms of ensuring stable operation and stability of the secondary battery. That is, if the temperature is the temperature range in the normal state of the secondary battery, the temperature increase at the relevant temperature adversely affects the performance of the secondary battery. The lower limit of the temperature at $R_n$ of Equation 2 above may be 70°C, 75°C, 80°C, 81°C, 82°C, 83°C, 84°C, 85°C, 86°C, 87°C, 88°C, 89°C, 90°C, 91°C, 92°C, 93°C, 94°C, or 95°C or so, and the upper limit thereof may be 200°C, 190°C, 180°C, 170°C, 160°C, 150°C, 140°C, 130°C, 120°C, 110°C, 100°C, or 90°C or so. The temperature may be within a range of more than, or equal to or more than any lower limit arbitrarily selected from the above-listed lower limits; or within a range of less than or equal to, or less than any upper limit arbitrarily selected from the above-listed upper limits while being more than or equal to, or more than any lower limit arbitrarily selected from the above-listed lower limits.

By controlling the resistance increase to occur in the temperature range, stable operation and storage in a normal state and stable blocking of energization in an abnormal state can be achieved. For example, through adjustment of the temperature range, stable storage is possible even when the secondary battery is stored at a relatively high temperature.

The polymer layer, and the current collector or the electrode, to which the polymer layer is applied, can exhibit a characteristic in which ΔR2 of Equation 3 below is in a certain level or more.

[Equation 3]

$$\Delta R2 = \mathrm{Max}\{(R_{z+5}/R_z)/5\}$$

In Equation 3, $R_z$ is the AC impedance resistance at an arbitrary temperature n°C within the range of 25°C to 135°C, $R_{z+5}$ is the AC impedance resistance at a temperature ((n+5)°C) that is 5°C higher than the temperature n°C, and Max{($R_{z+5}/R_z$)/5} is the maximum value among the ($R_{z+5}/R_z$)/5 values confirmed within the temperature range of 25°C to 135°C.

The method of measuring ΔR2 of Equation 3 above is described in the "9. Measurement of maximum resistance change rate (AC impedance)" section of Example sections. In the method for confirming ΔR2 above, the initial temperature is 25°C and the final temperature is 135°C. The AC impedance resistance is measured at each temperature while increasing the temperature by 5°C from the initial temperature of 25°C, thereby confirming the $R_{z+5}$ and $R_z$. For example, when n is 90, $R_{95}/R_{90}$ is the ratio of the AC impedance resistance at 95°C to the AC impedance resistance at 90°C. The matter that ΔR2 is 10 Ω/°C or more at any temperature within the temperature range of 25°C to 135°C means that the resistance of the polymer layer or electrode increases relatively rapidly at any temperature within the temperature range.

The lower limit of ΔR2 above may be 5, 10, 12, 14, 16, 18, 20, 22, 24, 26, 28, 30, or 33 or so, and the upper limit thereof may be 100, 95, 90, 85, 80, 75, 70, 65, 60, 55, 50, 45, 40, 35, 30, 25, or 20 or so. The unit of ΔR2 above is Ω/°C. The range of the ΔR2 may be within a range of more than, or equal to or more than any lower limit arbitrarily selected from the above-listed lower limits; or within a range of less than or equal to, or less than any upper limit arbitrarily selected from the above-listed upper limits while being more than or equal to, or more than any lower limit arbitrarily selected from the above-listed lower limits.

By securing the characteristics, it is possible to show an increase in resistance in an abnormal state, and by blocking energization therethrough, it is possible to ensure stability.

As in the case of the equation, the temperature at which the ΔR2 is confirmed, that is, the temperature range at $R_z$, is very important. The lower limit of the temperature may be 70°C, 75°C, 80°C, 81°C, 82°C, 83°C, 84°C, 85°C, 86°C, 87°C, 88°C, 89°C, 90°C, 91°C, 92°C, 93°C, 94°C, or 95°C or so, and the upper limit thereof may be 200°C, 190°C, 180°C, 170°C, 160°C, 150°C, 140°C, 130°C, 120°C, 110°C, 100°C, or 90°C or so. The temperature may be within a range of more than, or equal to or more than any lower limit arbitrarily selected from the above-listed lower limits; or within a range of less than or equal to, or less than any upper limit arbitrarily selected from the above-listed upper limits while being more than or equal to, or more than any lower limit arbitrarily selected from the above-listed lower limits.

By controlling the resistance increase to occur in the temperature range, stable operation and storage in a normal state and stable blocking of energization in an abnormal state can be possible. For example, through adjustment of the temperature range, stable storage is possible even when the secondary battery is stored at a relatively high temperature.

The polymer layer, the current collector or electrode to which the polymer layer is applied, or the secondary battery applied by the same can exhibit characteristics in which the absolute value of ΔR3 in Equation 4 below is within a certain range.

[Equation 4]

$$\Delta R3 = 100 \times (C_1 - C_2)/C_1$$

In Equation 4, $C_1$ is a discharge capacity at room temperature (about 25°C), and $C_2$ is a discharge capacity after storage at 70°C for 60 hours. $C_1$ and $C_2$ of Equation 4 are discharge capacities of the polymer layer measured for a coin cell, and a specific method of measuring the same is summarized in Examples.

The upper limit of the absolute value of ΔR3 in Equation 4 may be 10%, 9.5%, 9%, 8.5%, 8%, 7.5%, 7%, 6.5%, 6%, 5.5%, 5%, 4.5%, 4%, 3.5%, 3%, 2.5%, 2%, 1.5%, 1%, or 0.5% or so, and the lower limit thereof may be 0%, 0.5%, or 1.5% or so. The absolute value of ΔR3 may be within a range of less than or equal to, or less than any upper limit arbitrarily selected from the above-listed upper limits; or within a range of less than or equal to, or less than any upper limit arbitrarily selected from the above-listed upper limits while being more than or equal to, or more than any lower limit arbitrarily selected from the above-listed lower limits. ΔR3 of the above range means that stable operation and storage are possible even when the secondary battery is operated and stored at a relatively high temperature within the range of the normal state.

The polymer layer, the current collector or electrode to which the polymer layer is applied, or the secondary battery applied by the same may exhibit a characteristic in which the absolute value of ΔR4 in Equation 5 is 50% or more.

[Equation 5]

$$\Delta R4 = 100 \times (C_{25} - C_{130})/C_{25}$$

In Equation 5, $C_1$ is a discharge capacity at room temperature (about 25°C), and $C_3$ is a discharge capacity after storage at 130°C for 10 minutes.

$C_1$ and $C_3$ of Equation 5 are discharge capacities measured for a coin cell to which the polymer layer is applied, and a specific method of measuring the same is summarized in 11. High-temperature discharge capacity reduction rate of Example sections.

The lower limit of the absolute value of ΔR4 in Equation 5 may be 50%, 52%, 54%, 56%, 58%, 60%, 62%, 64%, 66%, 68%, 70%, 72%, 74%, 76%, 78%, 80%, 82%, 84%, 86%, or 88% or so, and the upper limit thereof may also be 200%, 180%, 160%, 140%, 120%, 100%, 95%, 90%, 85%, or 80% or so. The absolute value of ΔR4 may be within a range of more than or equal to, or more than any lower limit arbitrarily selected from the above-listed lower limits; or within a range of less than or equal to, or less than any upper limit arbitrarily selected from the above-listed upper limits while being more than or equal to, or more than any lower limit arbitrarily selected from the above-listed lower limits. ΔR4 in the above range means that stability is effectively secured by an increase in resistance in an abnormal state of the secondary battery.

The characteristics can be achieved through introduction of the polymer layer to be described below.

As the current collector, a current collector that is usually used as a current collector for a positive or negative electrode may be used.

If the current collector has conductivity without causing chemical changes in applied devices such as secondary batteries, the type, size, and shape thereof, and the like are not particularly limited. An example of a material that can be used as the current collector may be exemplified by a material, and the like in which the surface of copper, aluminum, or stainless steel is surface-treated with carbon, nickel, titanium, or silver, and the like. The current collector may be in the form of a film, sheet, foil, net, porous body, foam, or non-woven fabric, and the like, which comprises the above material. In some cases, a known surface treatment may also be performed on the surface of the current collector to improve adhesive force to other layers such as a polymer layer or an active material layer.

Such a current collector may typically have a thickness within a range of 3 μm to 500 μm, but is not limited thereto.

A polymer layer exists on one or both sides of the current collector.

The term polymer layer means a layer including a polymer. The lower limit of the polymer content in the polymer layer may be 30 wt%, 35 wt%, 40 wt%, 45 wt%, 50 wt%, 55 wt%, 60 wt%, 65 wt%, 70 wt%, 75 wt%, 80 wt%, 85 wt%, 90 wt%, or 95 wt% or so, and the upper limit thereof may be 100 wt%, 95 wt%, 90 wt%, 85 wt%, 80 wt%, 75 wt%, 70 wt%, 65 wt%, 60 wt%, 55 wt%, or 50 wt% or so. The content is the content of the polymer based on the total weight of the polymer layer. The content may be within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits.

The polymer layer may not be a so-called electrode active material layer. The content of the electrode active material in the polymer layer may be controlled. For example, the upper limit of the content of the electrode active material in the polymer layer may be 10 wt%, 9 wt%, 8 wt%, 7 wt%, 6 wt%, 5 wt%, 4 wt%, 3 wt%, 2 wt%, 1 wt%, 0.5 wt%, 0.1 wt%, 0.05 wt%, 0.01 wt%, 0.005 wt%, or 0.001 wt% or so, and the lower limit thereof may be 0 wt%. The content is the content of the electrode active material based on the total weight of the polymer layer. The content may be within a range of less than or equal to, or less than any upper limit arbitrarily selected from the above-listed upper limits; or within a range of more than or equal to, or more than the above-listed lower limit while being less than or equal to, or less than any upper limit arbitrarily selected from the above-listed upper limits.

The polymer layer may comprise a conductive polymer. As is known, the conductive polymer is a polymer exhibiting conductivity by a conjugated system of a polymer chain and/or doping, and the like. For example, the conductive polymer is a polymer exhibiting low resistance in a doping state and high resistance in a de-doping state, which may be a polymer designed so that the conversion between the doping and de-doping states may occur rapidly at a necessary time point in response to temperature and/or voltage.

The polymer layer may need to exhibit an appropriate level of oxidation potential depending on the application. For example, when the polymer layer is positioned between the current collector and the active material layer, as in the electrode, the polymer layer may need to exhibit a lower oxidation potential than the active material layer, and it is necessary for the low oxidation potential to be maintained even upon repeated charging/discharging and/or high-speed charging/discharging of the secondary battery. Otherwise, a potential drop phenomenon may occur during the process of the repeated charging/discharging and/or high-speed charging/discharging.

The thickness of the polymer layer may be appropriately controlled depending on the purpose. For example, the lower limit of the thickness of the polymer layer may be 10 nm, 50 nm, 100 nm, 150 nm, 200 nm, 250 nm, 300 nm, 350 nm, or 400 nm or so, and the upper limit thereof may be 2 μm, 1.5 μm, 1 μm, 950 nm, 900 nm, 850 nm, 800 nm, 750 nm, 700 nm, 650 nm, 600 nm, 550 nm, 500 nm, 450 nm, or 400 nm or so. The thickness may be within a range of less than or equal to, or less than any upper limit arbitrarily selected from the above-listed upper limits while being more than or equal to, or more than any lower limit arbitrarily selected from the above-listed lower limits.

The conductive polymer included in the polymer layer may be a thiophene polymer.

The term thiophene polymer means a polymer containing a thiophene monomer unit in a certain level or more.

For example, the lower limit of the ratio of the mole number of the thiophene monomer unit in the thiophene polymer may be 50 mol%, 55 mol%, 60 mol%, 65 mol%, 70 mol%, 75 mol%, or 80 mol% or so, and the upper limit thereof may be 100 mol%, 95 mol%, 90 mol%, or 80 mol% or so. The ratio of the mole number of the thiophene monomer unit is the ratio ($100\times M_T/M$) of the mole number ($M_T$) of all thiophene monomer units present in the thiophene polymer or the mole number (MT) of all thiophene monomers applied to prepare the thiophene polymer to the mole number (M) of all monomer units present in the thiophene polymer or the mole number (M) of all monomers applied to prepare the thiophene polymer. The ratio ($100\times M_T/M$) of the thiophene monomer unit may be within a range of more than or equal to, or more than any lower limit arbitrarily selected from the above-listed lower limits; or within a range of less than or equal to, or less than any upper limit arbitrarily selected from the above-listed upper limits while being more than or equal to, or more than any lower limit arbitrarily selected from the above-listed lower limits.

The term monomer unit means a form in which a certain monomer is polymerized and included in a polymer molecule (polymer). The term thiophene monomer is a thiophene-based monomer, which means a monomer containing a thiophene skeleton.

The conductive polymer may contain a long-chain hydrocarbon functional group or a monomer unit (hereinafter, referred to as unit A) having the long-chain hydrocarbon functional group. The unit A may be a thiophene monomer unit.

The term long-chain hydrocarbon functional group means a monovalent hydrocarbon group having a carbon number in a certain number or more, or a monovalent functional group containing the monovalent hydrocarbon group.

For example, the lower limit of the carbon number (i.e., the carbon number of the monovalent hydrocarbon group) present in the long-chain hydrocarbon functional group may be 3, 4, 5, 6, 7, 8, 9, 10, 11, or 12 or so, and the upper limit thereof may be 30, 29, 28, 27, 26, 25, 24, 23, 22, 21, 20, 19, 18, 17, 16, 15, 14, 13, 12, 11, 10, 9, 8, 7, 6, 5, or 4 or so. The carbon number may be within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits.

The carbon number may be the total carbon number present in the long-chain hydrocarbon functional group, or the carbon number of the hydrocarbon chain of the linear structure of the functional group. That is, the monovalent hydrocarbon group present in the long-chain hydrocarbon functional group may have a linear or branched structure. When the monovalent hydrocarbon group has the linear structure, the carbon number of the linear structure may be within the range. When the monovalent hydrocarbon group has the branched structure, the carbon number constituting the longest linear chain in the relevant branched structure may be within the range. For example, if the branched structure is a 2-ethylhexyl group, the carbon number constituting the longest linear chain is 6.

An example of the long-chain hydrocarbon functional group may be exemplified by one or more selected from the group consisting of an alkyl group, an alkenyl group, an alkynyl group, an alkoxy group, an alkylcarbonyl group, and an alkylcarbonyloxy group. In an appropriate example, the long-chain hydrocarbon functional group may be an alkyl group and/or an alkoxy group.

The carbon number present in the alkyl group, the alkenyl group, the alkynyl group, the alkoxy group, the alkyl group of the alkylcarbonyl group, and the alkyl group of the alkylcarbonyloxy group may be in the range of the carbon number (i.e., the carbon number of the monovalent hydrocarbon group) present in the long-chain hydrocarbon functional group.

The alkyl group, the alkenyl group, the alkynyl group, the alkoxy group, the alkyl group of the alkylcarbonyl group, and the alkyl group of the alkylcarbonyloxy group may be a linear or branched structure. In the case of a branched chain, the carbon number constituting the longest linear chain in the relevant branched structure may be in the range.

The alkyl group, the alkenyl group, the alkynyl group, the alkoxy group, the alkylcarbonyl group, or the alkylcarbonyloxy group, which is the long-chain hydrocarbon functional group, may also be optionally substituted with one or more substituents.

The long-chain hydrocarbon functional group may exhibit enhanced vibration energy at an increased temperature. This enhanced vibration energy may affect the doping and de-doping state of the conductive polymer. The degree of the vibration energy and the temperature at which the vibration energy appears are affected by the carbon number and arrangement state of the long-chain hydrocarbon functional group. Therefore, by adjusting the carbon number and amount of the long-chain hydrocarbon functional group and adjusting the arrangement state by the manufacturing method to be described below, it is possible to secure the desired properties for the polymer layer. For example, at the same temperature, the vibration energy of a relatively long chain is greater than that of a relatively short chain. Therefore, by appropriately employing a long chain and a short chain as the long-chain hydrocarbon functional group, it is possible to provide a conductive polymer to meet the intended effect.

Incidentally, the long-chain hydrocarbon functional group can provide appropriate mobility to a monomer or a polymer during the polymerization process of the conductive polymer, and accordingly, can also improve the polymerization efficiency.

For example, the ratio of the mole number ($M_L$) of the long-chain hydrocarbon functional group or the mole number ($M_L$) of the monomer unit (unit A) having the functional group to the mole number (M) of the total monomer units of the conductive polymer may be adjusted. For example, the lower limit of the ratio may be 20 mol%, 25 mol%, 30 mol%, 35 mol%, 40 mol%, 45 mol%, 50 mol%, 55 mol%, 60 mol%, 65 mol%, or 70 mol% or so, and the upper limit thereof may be 95 mol%, 90 mol%, 85 mol%, 80 mol%, or 75 mol% or so. The ratio is a ratio ($100 \times M_L/M$) of the mole number ($M_L$) of all long-chain hydrocarbon functional groups present in the conductive polymer or the mole number ($M_L$) of monomer units having the long-chain hydrocarbon functional group or the mole number ($M_L$) of monomers having all long-chain hydrocarbon functional groups applied to prepare the thiophene polymer to the mole number (M) of all monomer units present in the conductive polymer or the mole number (M) of all monomers applied to prepare the conductive polymer. The ratio may be within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits.

The conductive polymer may include a first hydrocarbon functional group and a second hydrocarbon functional group.

The first hydrocarbon functional group is a functional group having a relatively large carbon number among the long-chain hydrocarbon functional groups. The lower limit of the carbon number of the first hydrocarbon functional group may be 10, 11, or 12 or so, and the upper limit thereof may also be 20, 19, 18, 17, 16, 15, 14, 13, 12, 11, or 10 or so. The carbon number of the first hydrocarbon functional group may be within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits.

The second hydrocarbon functional group is a functional group having a relatively small carbon number among the long-chain hydrocarbon functional groups. The lower limit of the carbon number of the second hydrocarbon functional group may be 3, 4, 5, 6, 7, or 8 or so, and the upper limit thereof may also be 9, 8, 7, or 6 or so. The carbon number of the second hydrocarbon functional group may be within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits.

The carbon number of each of the first and second hydrocarbon functional groups may be the carbon number of the hydrocarbon chain of the linear structure present in the hydrocarbon functional group. For example, the first and second hydrocarbon functional groups may each independently be one or more selected from the group consisting of an alkyl group, an alkenyl group, an alkynyl group, an alkoxy group, an alkylcarbonyl group, and an alkylcarbonyloxy group,

and may be, in a suitable example, an alkyl group and/or an alkoxy group, where the carbon number may be the carbon number of the alkyl group, the alkenyl group, the alkynyl group, the alkoxy group, the alkyl group of the alkylcarbonyl group, and the alkyl group of the alkylcarbonyloxy group.

The alkyl group, the alkenyl group, the alkynyl group, the alkoxy group, the alkyl group of the alkylcarbonyl group, and the alkyl group of the alkylcarbonyloxy group may have a linear or branched structure, and in the case of the linear chain, the total carbon number may be in the range, and in the case of the branched chain, the carbon number constituting the longest linear chain in the branched structure may be in the range.

As described above, the carbon number of the long-chain hydrocarbon functional group is a functional group exhibiting enhanced vibration energy at an increased temperature, where the higher the carbon number at the same temperature, the higher the vibration energy. That is, the first hydrocarbon functional group exhibits higher vibration energy than the second hydrocarbon functional group at the same temperature, and the sum of the vibration energies of these two functional groups may optimize the properties of the conductive polymer that are difficult to precisely control with one functional group.

For example, the first hydrocarbon functional group exhibits enhanced vibration energy at an increased temperature, and the second hydrocarbon functional group dilutes the vibration energy by the first hydrocarbon functional group at the same temperature, whereby it is possible to precisely control the start temperature (onset point) of the de-doping of the conductive polymer according to the purpose.

The ratio of the sum mole number of the first and second hydrocarbon functional groups or the sum mole number of the monomer units having the first hydrocarbon functional group and the monomer units having the second hydrocarbon functional group relative to the mole number (M) of the total monomer units of the conductive polymer may adjusted in the same range as the ratio $100\times M_L/M$ as described above.

The lower limit of the ratio ($M_2/M_1$) of the mole number ($M_2$) of the second hydrocarbon functional group or the mole number ($M_2$) of the monomer units having the second hydrocarbon functional group to the mole number ($M_1$) of the first hydrocarbon functional group or the mole number ($M_1$) of the monomer units having the first hydrocarbon functional group may be 0.01, 0.05, 0.1, or 0.5 or so, and the upper limit thereof may be 100, 95, 90, 85, 80, 75, 70, 65, 60, 55, 50, 45, 40, 35, 30, 25, 20, 15, 10, 9, 8, 7, 6, 5, 4, 3, 2, 1, or 0.7 or so. The ratio may be within a range of more than or equal to, or more than any lower limit arbitrarily selected from the above-listed lower limits while being less than or equal to, or less than any upper limit arbitrarily selected from the above-listed upper limits. The ratio $M_2/M_1$ may be changed in consideration of the level of the desired vibrational energy and the design value of the polymer layer.

The conductive polymer may contain a polar functional group together with the long-chain hydrocarbon functional group. The monomer having the polar functional group may be a thiophene monomer.

The term polar functional group is a functional group containing one, or two or more polar atoms, for example, oxygen and/or nitrogen. An example of such a functional group includes a carboxyl group, a hydroxyl group, an amino group, a cyano group, a nitro group, an ether group, an alkoxy group, and/or a functional group of Formula 1 below, but is not limited thereto.

Here, the alkoxy group may be, in one example, an alkoxy group with 1 to 20 carbon atoms, 1 to 16 carbon atoms, 1 to 12 carbon atoms, 1 to 8 carbon atoms or 1 to 4 carbon atoms. The alkoxy group may be linear, branched, or cyclic, and may be linear or branched, as appropriate. The alkoxy group may be optionally substituted with one or more substituents.

In one example, the polar functional group may be a functional group of Formula 1 below.

[Formula 1]

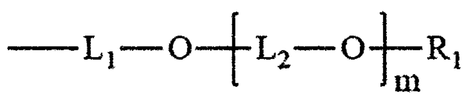

In Formula 1, $L_1$ is a single bond, an alkylene group or an alkylidene group, $L_2$ is an alkylene group or an alkylidene group, $R_1$ is hydrogen or an alkyl group, and m is an arbitrary number.

In Formula 1, the matter that $L_1$ is a single bond means a form that $L_1$ does not exist and the oxygen atom between $L_1$ and $L_2$ is directly connected to the monomer.

In one example, the alkyl group of $R_1$ in Formula 1 may be an alkyl group with 1 to 20 carbon atoms, 1 to 16 carbon atoms, 1 to 12 carbon atoms, 1 to 8 carbon atoms or 1 to 4 carbon atoms, or may be a methyl group or an ethyl group. The alkyl group may be linear, branched, or cyclic, and may be linear or branched, as appropriate. The alkyl group may be optionally substituted with one or more substituents.

The term alkylene group means a divalent functional group formed by removing one hydrogen atom from each of two different carbon atoms of an alkane, and the term alkylidene group means a divalent functional group formed by removing two hydrogen atoms from one carbon atom of an alkane.

In one example, the alkylene groups of $L_1$ and $L_2$ in Formula 1 may each be an alkylene group with 2 to 20 carbon

atoms, 2 to 16 carbon atoms, 2 to 12 carbon atoms, 2 to 8 carbon atoms or 2 to 4 carbon atoms, or may each be an ethylene group or a propylene group. The alkylene group may be linear, branched, or cyclic, and may be linear or branched, as appropriate. The alkylene group may be optionally substituted with one or more substituents.

In one example, the alkylidene groups of $L_1$ and $L_2$ in Formula 1 may each be an alkylidene group with 1 to 20 carbon atoms, 1 to 16 carbon atoms, 1 to 12 carbon atoms, 1 to 8 carbon atoms or 1 to 4 carbon atoms, or may each be a methylidene group, an ethylene group or a propylidene group. The alkylidene group may be linear, branched, or cyclic, and may be linear or branched, as appropriate. The alkylidene group may be optionally substituted with one or more substituents.

In Formula 1, the lower limit of m may be 1, 2, 3, or 4, and the upper limit thereof may be 10, 9, 8, 7, 6, 5, 4, or 3 or so. The above n may be within a range of more than or equal to, or more than any lower limit arbitrarily selected from the above-listed lower limits while being less than or equal to, or less than any upper limit arbitrarily selected from the above-listed upper limits.

The polar functional group may bind the polymer layer to another layer to have an appropriate bonding force, and may significantly improve dispersibility of a conductive material, which is described below, in the polymer layer in combination with the long-chain hydrocarbon functional group. In addition, the polar functional group may also play a role in suppressing the PTC effect from appearing at a relatively low temperature.

The mole numbers of the polar functional group and the long-chain hydrocarbon functional group in the conductive polymer may be controlled to ensure an appropriate effect.

For example, the lower limit of the ratio ($M_L/M_P$) of the mole number ($M_L$) of the long-chain hydrocarbon functional group or the mole number ($M_L$) of the monomer units having the long-chain hydrocarbon functional group to the mole number ($M_L$) of the polar functional group or the mole number ($M_L$) of the monomer units having the polar functional group in the conductive polymer may be 1, 1.5, 2, 2.5, 3, 3.5, 4, 4.5, 5, 5.5, 6 6.5, 7, 7.5, 8, 8.5, or 9 or so, and the upper limit thereof may be 500, 450, 400, 350, 300, 250, 200, 150, 100, 90, 80, 70, 60, 50, 40, 30, 20, 19, 18, 17, 16, 15, 14, 13, 12, 11, 10, or 9.5 or so. The ratio $M_L/M_P$ may be within a range of less than or equal to, or less than any upper limit arbitrarily selected from the above-listed upper limits; or within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits. Here, when the ratio $M_L/M_P$ is a ratio between monomer units, the relevant ratio may be a ratio of the mole number $M_L$ of monomer units having all long-chain hydrocarbon functional groups present in the conductive polymer to the mole number $M_P$ of monomer units having all polar functional groups; or a ratio of the mole number $M_L$ of monomers having all long-chain hydrocarbon functional groups applied to manufacture the conductive polymer to the mole number $M_P$ of monomers having all polar functional groups.

The ratio of the sum mole number $M_S$ of the monomer units having the long-chain hydrocarbon functional group and the monomer units having the polar functional group to the mole number M of all monomer units in the conductive polymer may be adjusted within the same range as the ratio $100 \times M_T/M$ as described above. In this instance, the mole number $M_S$ is substituted instead of the mole number $M_T$.

For example, the conductive polymer may contain a unit of Formula 2 below as the thiophene monomer unit.

[Formula 2]

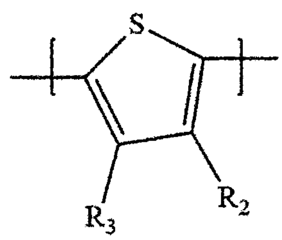

In Formula 2, $R_2$ and $R_3$ may each independently be hydrogen, the polar functional group, or the long-chain hydrocarbon functional group. In another example, $R_2$ and $R_3$ of Formula 2 may also be connected to each other to form a divalent functional group of Formula 3.

[Formula 3]

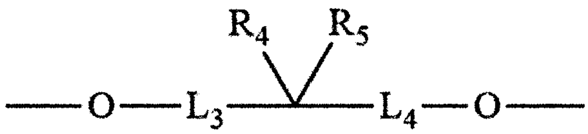

In Formula 3, $L_3$ and $L_4$ may each independently be a single bond, an alkylene group, or an alkylidene group, and $R_4$ and $R_5$ may each independently be hydrogen, the polar functional group, or the long-chain hydrocarbon functional group.

In Formula 2, when $R_2$ and $R_3$ are each independently hydrogen, a polar functional group, or a long-chain hydrocarbon functional group, at least one of $R_2$ and $R_3$ above is the polar functional group or the long-chain hydrocarbon functional group.

In Formula 2, when $R_2$ and $R_3$ form the divalent functional group of Formula 3, at least one of $R_4$ and $R_5$ above is the polar functional group or the long-chain hydrocarbon functional group.

In Formula 3, the meanings of the single bond, alkylene group, and alkylidene group, and specific examples are the same as in Formula 1. The technical significance and specific examples of the long-chain hydrocarbon functional group and polar functional group in Formulas 2 and 3 are as described above.

The ratio of the mole number $M_{C2}$ of the unit of Formula 2 to the mole number M of the total monomer units of the conductive polymer may be adjusted to be the same as the ratio $100 \times M_T/M$, where $M_T$ is replaced by $M_{C2}$.

In one example, the conductive polymer may contain a monomer unit represented by Formula 4 below. The monomer unit of Formula 4 above may be one example of the monomer units having the first hydrocarbon functional group.

[Formula 4]

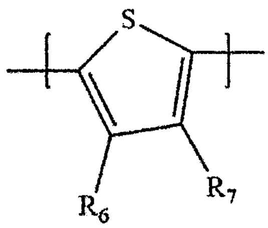

In Formula 4, $R_6$ and $R_7$ may each independently be hydrogen or the first hydrocarbon functional group. In this case, one or more of $R_6$ and $R_7$ above is the first hydrocarbon functional group.

In another example, $R_6$ and $R_7$ above may be connected to each other to form a divalent functional group of Formula 5.

[Formula 5]

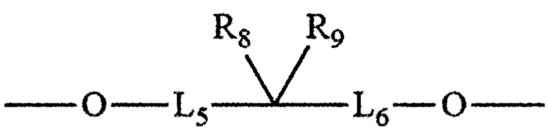

In Formula 5, $L_5$ and $L_6$ are each independently a single bond, an alkylene group, or an alkylidene group, and $R_8$ and $R_9$ are each independently hydrogen or the first hydrocarbon functional group, but at least one of $R_8$ and $R_9$ is the first hydrocarbon functional group.

The specific details of the first hydrocarbon functional group are as described above, and the specific details of the single bond, alkylene group, or alkylidene group are as described in Formula 1 above.

The conductive polymer may also contain a monomer unit represented by Formula 6 below. The monomer unit of Formula 6 above may be one example of the monomer units having the second hydrocarbon functional group.

[Formula 6]

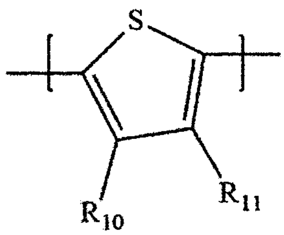

In Formula 6, $R_{10}$ and $R_{11}$ may each independently be hydrogen or the second hydrocarbon functional group, and in this case, one or more of $R_{10}$ and $R_{11}$ above is the second hydrocarbon functional group.

In another example, $R_{10}$ and $R_{11}$ above may be connected to each other to form a divalent functional group of Formula 7 below.

[Formula 7]

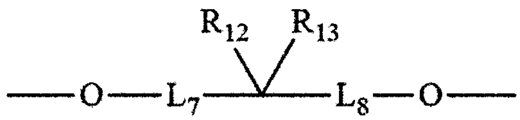

In Formula 7, $L_7$ and $L_8$ are each independently a single bond, an alkylene group, or an alkylidene group, and $R_{12}$ and $R_{13}$ are each independently hydrogen or the second hydrocarbon functional group, but at least one of $R_{12}$ and $R_{13}$ is the second hydrocarbon functional group.

The specific details of the second hydrocarbon functional group are as described above, and the specific details of the single bond, the alkylene group, and the alkylidene group are as described in Formula 1.

The conductive polymer may also contain a monomer unit represented by Formula 8 below. The monomer unit of Formula 8 above may be one example of the monomer units having the polar functional group.

[Formula 8]

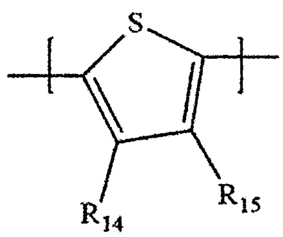

In Formula 8, $R_{14}$ and $R_{15}$ may each independently be hydrogen or the polar functional group. In the above case, one or more of $R_{14}$ and $R_{15}$ above are the polar functional group.

In another example, $R_{14}$ and $R_{15}$ of Formula 8 above may be connected to each other to form a divalent functional group of Formula 9 below.

[Formula 9]

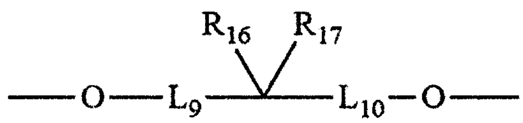

In Formula 9, $L_9$ and $L_{10}$ are each independently a single bond, an alkylene group, or an alkylidene group, and $R_{16}$ and $R_{17}$ are each independently hydrogen or a polar functional group, but at least one of $R_{16}$ and $R_{17}$ is the polar functional group.

The specific details of the polar functional group are as described above, and the specific details of the single bond, alkylene group, and alkylidene group are as described in Formula 1.

In the case where the conductive polymer simultaneously contains the monomer unit of Formula 4 above and the monomer unit of Formula 6 above, the ratio of the sum mole number $M_{4+6}$ of the monomer unit of Formula 4 above and the

monomer unit of Formula 6 above to the mole number M of the total monomer units included in the conductive polymer may be adjusted within the same range as the ratio $100 \times M_L/M$ as described above, where $M_L$ is replaced by $M_{4+6}$.

The ratio of the mole number $M_4$ of the monomer unit of Formula 4 above and the mole number $M_6$ of the monomer unit of Formula 6 above may be adjusted within the same range as the ratio $M_2/M_1$. At this time, the mole number $M_1$ may be the mole number of the monomer unit of Formula 4 above, and the mole number $M_2$ may be the mole number of the monomer unit of Formula 6 above.

When the conductive polymer contains the monomer unit of Formula 8 above, the unit may be included so that the molar ratio $M_L/M_P$ is satisfied. In this instance, the mole number of the monomer unit of Formula 8 above becomes the mole number $M_P$. In addition, the mole number $M_L$ may be the mole number of the monomer unit of Formula 4 above, the mole number of the monomer unit of Formula 6 above, or the sum mole number of the monomer unit of Formula 4 above and the monomer unit of Formula 6 above.

When the monomer units of Formulas 4, 6, and 8 above are present in the conductive polymer, the ratio of the sum mole number $M_{4+6+8}$ of the monomer units of Formulas 4, 6, and 8 above to the sum mole number M of all monomer units in the conductive polymer may be adjusted within the same range as the ratio $100 \times M_T/M$. In this instance, the mole number $M_{4+6+8}$ becomes the mole number $M_T$.

The conductive polymer contains the monomer units, which may further contain other monomer units. For example, the conductive polymer may contain a no-thiophene monomer unit in addition to the thiophene monomer unit.

The no-thiophene monomer unit is a unit of a monomer other than a thiophene-based monomer, which is a unit for adjusting the oxidation potential of the conductive polymer. For example, the no-thiophene monomer unit may be a unit of a monomer which is copolymerizable with the thiophene monomer and exhibits a lower oxidation potential than polythiophene when manufactured as a homopolymer.

In the above case, the molar ratio of the thiophene monomer unit in the conductive polymer may be adjusted within the same range as the ratio $100 \times M_T/M$. In addition, the ratio of the no-thiophene monomer unit may be adjusted in consideration of the desired oxidation potential.

For example, in the conductive polymer, the lower limit of the mole number of the no-thiophene monomer unit per 1 mole of the thiophene monomer unit may be 0.05 mol, 0.1 mol, 0.15 mol, 0.2 mol, 0.22 mol, or 0.24 mol or so, and the upper limit thereof may be 1 mol, 0.9 mol, 0.8 mol, 0.7 mol, 0.6 mol, 0.5 mol, 0.4 mol, or 0.3 mol or so. The ratio may be within a range of less than or equal to, or less than any upper limit arbitrarily selected from the above-listed upper limits; or within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits.

The no-thiophene monomer unit may be, for example, an aromatic monomer unit or a nitrogen-containing heterocyclic monomer unit. The aromatic monomer unit is a unit formed from an aromatic monomer unit, and the nitrogen-containing heterocyclic monomer unit is a unit formed from a nitrogen-containing heterocyclic monomer.

For example, the no-thiophene monomer unit may be a monomer unit of Formula 10 below.

[Formula 10]

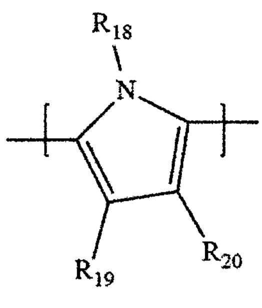

In Formula 10, $R_{18}$, $R_{19}$, and $R_{20}$ may each independently be hydrogen, the polar functional group, or the hydrocarbon functional group.

An example of the hydrocarbon functional group includes an alkyl group, an alkenyl group, or an aryl group.

The alkyl groups may each independently be an alkyl group with 1 to 20 carbon atoms, 1 to 16 carbon atoms, 1 to 12 carbon atoms, 1 to 8 carbon atoms, or 1 to 4 carbon atoms.

The alkenyl group may be an alkenyl group with 2 to 20 carbon atoms, 2 to 16 carbon atoms, 2 to 12 carbon atoms, 2 to 8 carbon atoms, or 2 to 4 carbon atoms.

The aryl group may be, for example, an aryl group with 6 to 30 carbon atoms, or 6 to 26 carbon atoms, or 6 to 22 carbon atoms, or 6 to 20 carbon atoms, or 6 to 18 carbon atoms, or 6 to 15 carbon atoms.

The alkyl group and the alkenyl group may each independently be linear, branched, or cyclic. The alkyl group, the alkenyl group, and the aryl group may each independently optionally be substituted with one or more substituents.

The specific details of the polar functional group are as described above. For example, the unit of Formula 10 above may be a polypyrrole unit.

In another example, the no-thiophene monomer unit may be a unit of Formula 11 below.

[Formula 11]

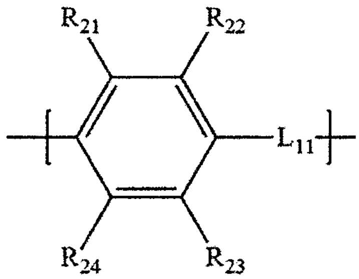

In Formula 11, $L_{11}$ may be a single bond, an alkylene group, an alkylidene group, O or $NR^1$. Here, the meaning of the single bond, the alkylene group or the alkylidene group is as described in Formula 1. In addition, the $R^1$ may be hydrogen, an alkyl group, an alkenyl group, or an aryl group.

In Formula 11, $R_{21}$ to $R_{24}$ may each independently be hydrogen, an alkyl group, an alkenyl group, an amino group, an alkoxy group, an aryl group, or a halogen atom.

The specific types of the alkyl group, the alkenyl group, and the aryl group in Formula 11 are as described in Formula 10.

The alkoxy group in Formula 11 may be an alkoxy group with 1 to 20 carbon atoms, 1 to 16 carbon atoms, 1 to 12 carbon atoms, 1 to 8 carbon atoms, or 1 to 4 carbon atoms. The alkoxy groups may each independently be linear, branched, or cyclic. The alkoxy group may be optionally substituted with one or more substituents.

For example, the monomer unit in Formula 11 may be a polyaniline or polyphenylene monomer unit.

In another example, the no-thiophene monomer unit may be a unit of Formula 12 below.

[Formula 12]

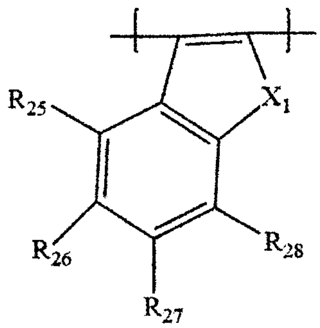

In Formula 12, $X_1$ may be S, O or $NR^1$. In addition, $R^1$ may be hydrogen, an alkyl group, an alkenyl group, or an aryl group.

In Formula 12, $R_{25}$ to $R_{28}$ may each independently be hydrogen, an alkyl group, an alkenyl group, an alkoxy group, an aryl group, or halogen.

The specific types of the alkyl group, the alkenyl group, the alkoxy group and the aryl group in Formula 12 are the same as in Formulas 10 and 11. The monomer unit of Formula 12 may be a polyindole unit.

In another example, the no-thiophene monomer unit may be a unit of Formula 13 below.

[Formula 13]

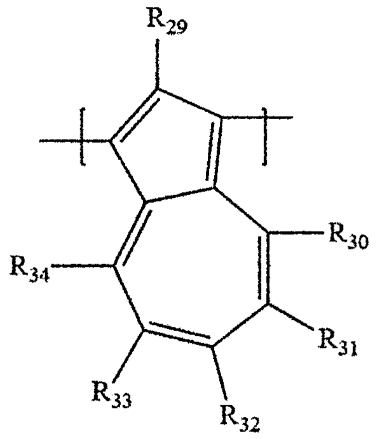

In Formula 13, $R_{29}$ to $R_{34}$ may each independently be hydrogen, an alkyl group, an alkenyl group, an alkoxy group, an aryl group, a hydroxy group, or halogen. The specific types of the alkyl group, alkenyl group, alkoxy group, and aryl group in Formula 13 are the same as in Formulas 10 and 11.
The monomer unit of Formula 13 above may be a polyazulene unit.
In another example, the no-thiophene monomer unit may be a unit of Formula 14 below.

[Formula 14]

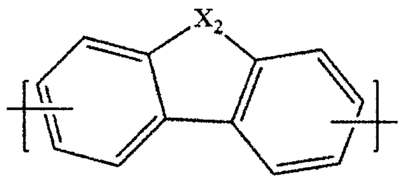

In Formula 14, $X_2$ may be a carbon atom ($CR^aR^b$) or a nitrogen atom ($NR^1$). Here, $R^a$, $R^b$, and $R^1$ may each independently be hydrogen, an alkyl group, an alkenyl group, or an aryl group.
Meanwhile, the unit of Formula 14 above may be unsubstituted or substituted with one or more selected from the group consisting of an alkyl group, an alkenyl group, an alkoxy group, an aryl group, and halogen.
The specific types of the alkyl group, the alkenyl group, the alkoxy group, and the aryl group in Formula 14 are the same as in Formulas 10 and 11.
For example, the monomer unit of Formula 14 above may be a polyfluorene or polycarbazole unit.
In another example, the no-thiophene monomer unit may be a unit of Formula 15 below.

[Formula 15]

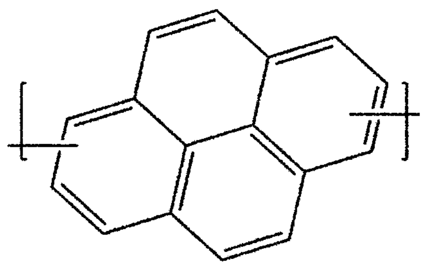

The unit of Formula 15 may be unsubstituted or substituted with one or more selected from the group consisting of an alkyl group, an alkenyl group, an alkoxy group, an aryl group, and halogen.
The specific types of the alkyl group, alkenyl group, alkoxy group and aryl group in Formula 15 above are the same as in Formulas 10 and 11. For example, the monomer unit of Formula 15 above may be a polypyrene unit.
The conductive polymer may have a weight average molecular weight in a predetermined range. The lower limit of the weight average molecular weight of the conductive polymer may be 10,000, 15,000, 20,000, 25,000, 30,000, 35,000, 40,000, 45,000, 50,000, 55,000, 60,000, 65,000, 70,000, 75,000, 80,000, 85,000, 90,000, or 95,000 or so, and the upper

limit thereof may also be 1,000,000, 950,000, 900,000, 850,000, 800,000, 750,000, 700,000, 650,000, 600,000, 550,000, 500,000, 450,000, 400,000, 350,000, 300,000, 250,000, 200,000, 150,000, 130,000, 110,000, or 100,000 or so. The weight average molecular weight may be within a range of less than or equal to, or less than any upper limit arbitrarily selected from the above-listed upper limits while being more than or equal to, or more than any lower limit arbitrarily selected from the above-listed lower limits. The unit of the weight average molecular weight is g/mol, which may be confirmed according to the description of "2. GPC (Gel Permeation Chromatograph)" of Example sections of this specification.

The molecular weight distribution of the conductive polymer, i.e., the ratio (Mw/Mn) of the weight average molecular weight (Mw) to the number average molecular weight (Mn) may be within a predetermined range. The lower limit of the molecular weight distribution may be 2, 2.5, 3, or 3.5 or so, and the upper limit thereof may be 8, 7.5, 7, 6.5, 6, 5.5, 5, 4.5, 4, or 3.7 or so. The molecular weight distribution may have a range of less than or equal to, or less than any upper limit arbitrarily selected from the above-listed upper limits while being more than or equal to, or more than any lower limit arbitrarily selected from the above-listed lower limits. The molecular weight distribution may be confirmed according to the description of "2. GPC (Gel Permeation Chromatograph)" of Example sections of this specification.

The conductive polymer may further contain other monomer units if it contains the above-described units in the above ratio.

The polymer layer comprises the conductive polymer, and accordingly, may exhibit the above-described characteristics. The polymer layer may also comprise any additional component if it comprises the conductive polymer.

For example, the polymer layer may further comprise a conductive material together with the conductive polymer. Through addition of such a material, the oxidation potential of the polymer layer may be further adjusted. As the conductive material, a material having appropriate conductivity may be used, and for example, as the conductive material one, or two or more selected from carbon particles, carbon fibers, graphene, graphite, carbon black, and carbon nanotubes, and the like may be used.

As the conductive material, an appropriate type may be selected and used from the types as described above, and the shape of the material may be a particle shape (spherical, irregular, or other shape), a plate shape, or a fiber shape, and the like, but is not limited thereto.

The size of the conductive material may also be appropriately adjusted as needed. For example, the lower limit of the size of the conductive material may be 10 nm, 50 nm, 100 nm, 150 nm, 200 nm, 250 nm, 300 nm, 350 nm, 400 nm, 450 nm, 500 nm, 550 nm, 600 nm, 650 nm, 700 nm, 750 nm, 800 nm, 850 nm, 900 nm, 950 nm, 1000 nm, 5000 nm, or 10000 nm or so, and the upper limit thereof may also be 100000 nm, 90000 nm, 80000 nm, 70000 nm, 60000 nm, 50000 nm, 40000 nm, 30000 nm, 20000 nm, 10000 nm, 5000 nm, 1000 nm, 950 nm, 900 nm, 850 nm, 800 nm, 750 nm, 700 nm, 650 nm, 600 nm, 550 nm, 500 nm, 450 nm, 400 nm, 350 nm, 300 nm, 250 nm, 200 nm, 200 nm, 150 nm, 100 nm, 90 nm, 80 nm, or 70 nm or so. The size may be within a range of less than or equal to, or less than any upper limit arbitrarily selected from the above-listed upper limits; or within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits. The above size is the average diameter (so-called D50 particle size) measured according to the contents of "12. Average particle diameter" section described in Example sections of this specification.

If necessary, the conductive material may be surface-treated considering dispersibility, and the like.

In this case, a surface-treating agent having appropriate compatibility with the conductive polymer may be used as the surface-treating agent. For example, the conductive material may be surface-treated with a polyphenol-based compound as the surface-treating agent. The polyphenol-based compound means a compound including a structure containing two or more hydroxyl groups which are substituted in benzene, and connected thereto. Such a compound may be exemplified by a so-called catechol compound (i.e., catechol, or compound including the relevant structure), and an example thereof includes dopamine, polydopamine, 3,4-dihydroxy phenyl alanine, norepinephrine, tannic acid, humic acid, and/or lignin, and the like, but is not limited thereto.

The method of surface-treating the conductive material with the surface-treating agent is not limited, and for example, a method of mixing the conductive material and the surface-treating agent in an appropriate solvent, and the like, or a method of synthesizing or polymerizing the surface-treating agent on the surface of the conductive material may be applied.

The content of the conductive material may be adjusted in consideration of the desired oxidation potential. In general, as the content of the conductive material in the polymer layer increases, the oxidation potential of the polymer layer decreases. Therefore, the content of the conductive material may be adjusted in consideration of the oxidation potential of the electrode active material and accordingly, the required oxidation potential of the polymer layer. For example, the lower limit of the content of the conductive material in the polymer layer, relative to 100 parts by weight of the conductive polymer, may be 0.5 parts by weight, 1 part by weight, 5 parts by weight, 10 parts by weight, 15 parts by weight, 20 parts by weight, 30 parts by weight, 40 parts by weight, 50 parts by weight, 60 parts by weight, 70 parts by weight, 80 parts by weight, 90 parts by weight, or 100 parts by weight or so, and the upper limit thereof may be 1,000 parts by weight,

900 parts by weight, 800 parts by weight, 700 parts by weight, 600 parts by weight, 500 parts by weight, 400 parts by weight, 300 parts by weight, 200 parts by weight, 150 parts by weight, 100 parts by weight, 50 parts by weight, 48 parts by weight, 46 parts by weight, 44 parts by weight, 42 parts by weight, 40 parts by weight, 38 parts by weight, 36 parts by weight, 34 parts by weight, 32 parts by weight, 30 parts by weight, 28 parts by weight, 26 parts by weight, 24 parts by weight, 22 parts by weight, 20 parts by weight, 18 parts by weight, 16 parts by weight, 14 parts by weight, 12 parts by weight, or 10 parts by weight or so. The content may be within a range of less than or equal to, or less than any upper limit arbitrarily selected from the above-listed upper limits; or within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits.

Under such a ratio, the conductive material appropriately interacts with the conductive polymer, whereby it is possible that a polymer layer in a desired shape may be effectively formed.

The electrode may comprise an active material layer formed on the polymer layer. A commonly applied layer may also be used as the active material layer.

Typically, the active material layer comprises an electrode active material. The specific type of the electrode active material is not particularly limited, and materials to form a positive electrode or a negative electrode may usually be used.

For example, when the active material layer is a positive electrode active material layer, the electrode active material may include layered compounds such as lithium cobalt oxide ($LiCoO_2$) or lithium nickel oxide ($LiNiO_2$), or compounds substituted with one or more transition metals; lithium iron oxides such as $LiFe_3O_4$; lithium manganese oxides such as Formula $Li_{1+c1}Mn_{2-c1}O_4$ ($0 \leq c1 \leq 0.33$), $LiMnO_3$, $LiMn_2O_3$, or $LiMnO_2$; lithium copper oxides ($Li_2CuO_2$); vanadium oxides such as $LiV_3O_8$, $V_2O_5$, or $Cu_2V_2O_7$; Ni-site lithium nickel oxides represented by Formula $LiNi_{1-c2}M_{c2}O_2$ (where M is at least one selected from the group consisting of Co, Mn, Al, Cu, Fe, Mg, B and Ga, and $0.01 \leq c2 \leq 0.3$ is satisfied); lithium manganese composite oxides represented by Formula $LiMn_{2-c3}M_{c3}O_2$ (where M is at least one selected from the group consisting of Co, Ni, Fe, Cr, Zn, and Ta, and $0.01 \leq c3 \leq 0.1$ is satisfied) or $Li_2Mn_3MO_8$ (here, M is at least one selected from the group consisting of Fe, Co, Ni, Cu and Zn); lithium nickel cobalt manganese (NCM) composite oxides, lithium nickel cobalt manganese aluminum (NCMA) composite oxides, and $LiMn_2O_4$ in which a part of Li in the formula is substituted with alkaline earth metal ions, and the like, but is not limited thereto.

When the active material layer is a negative electrode active material layer, as the electrode active material, for example, a compound capable of reversible intercalation and deintercalation of lithium may be used. A specific example may include carbonaceous materials such as artificial graphite, natural graphite, graphitized carbon fiber, and amorphous carbon; metallic compounds capable of alloying with lithium, such as Si, Al, Sn, Pb, Zn, Bi, In, Mg, Ga, Cd, Si alloy, Sn alloy, or Al alloy; metal oxides capable of doping and de-doping lithium, such as $SiO_a$ ($0 < a < 2$), $SnO_2$, vanadium oxide, and lithium vanadium oxide; or a composite comprising the metallic compound and the carbonaceous materials such as a Si-C composite or a Sn-C composite, and the like, and any one or a mixture of two or more of the foregoing may be used.

A metal lithium thin film may also be used as the negative electrode active material, and as the carbon material, low crystalline carbon and high crystalline carbon, and the like may also be used. As the low crystalline carbon, soft carbon and hard carbon are representative, and as the high crystalline carbon, high-temperature baked carbons such as amorphous, plate-like, scale-like, spherical, or fibrous natural graphite or artificial graphite, Kish graphite, pyrolytic carbon, mesophase pitch based carbon fiber, mesocarbon microbeads, mesophase pitches, and petroleum and coal tar pitch derived cokes are representative.

The electrode active material may be included in the active material layer within a range of about 80 wt% to 99.5 wt%, or within a range of 88 wt% to 99 wt%, relative to the total weight of the active material layer, but the ratio may be changed depending on the use or design of the electrode.

The active material layer may further comprise a binder. The binder serves to improve attachment between the active materials, and adhesive force on between the active material layer and the current collector. An example of the binder is not particularly limited, and one or more may be selected from the group consisting of, for example, PVDF (poly(vinylidene fluoride), PVA (poly(vinyl alcohol)), SBR (styrene butadiene rubber), PEO (poly(ethylene oxide)), CMC (carboxyl methyl cellulose), cellulose acetate, cellulose acetate butylate, cellulose acetate propionate, cyanoethylpullulan, cyanoethyl polyvinyl alcohol, cyanoethyl cellulose, cyanoethyl sucrose, pullulan, polymethylmethacrylate, polybutylacrylate, polyacrylonitrile, polyvinylpyrrolidone, polyvinylacetate, an ethylene vinyl acetate polymer (polyethylene-co-vinyl acetate), and polyarylate, and used.

In one example, the binder may be included in the active material layer within a range of 0.1 parts by weight to 10 parts by weight or 0.5 parts by weight to 5 parts by weight, relative to 100 parts by weight of the electrode active material, but is not limited thereto.

The active material layer may further comprise a conductive material, as needed. If the conductive material has conductivity without causing chemical changes in the secondary battery, it is not particularly limited, any known material can be used. For example, graphite such as natural graphite or artificial graphite; carbon black, such as carbon black,

acetylene black, Ketjen black, channel black, furnace black, lamp black, or thermal black; conductive fibers such as carbon fibers or metal fibers; conductive tubes such as carbon nanotubes (CNTs); metal powders such as fluorocarbon, aluminum, or nickel powder; conductive whiskers such as zinc oxide or potassium titanate; conductive metal oxides such as titanium oxide; conductive materials such as polyphenylene derivatives, and the like may be used.

In one example, the conductive material may be included in the active material layer in an amount of 0.1 parts by weight to 20 parts by weight or 0.3 parts by weight to 10 parts by weight, relative to 100 parts by weight of the electrode active material, but is not limited thereto.

The active material layer may also further comprise any necessary known components in addition to the above-described components optionally.

As described above, in order that the polymer layer exhibits the desired effect, selection of the long-chain hydrocarbon functional group and/or the conductive material is important, and additionally, control of the arrangement state of the long-chain hydrocarbon functional group and/or the dispersion state of the conductive material is important, where the desired arrangement and/or dispersion state may be secured by a manufacturing method to be described below.

The manufacturing method may comprise a step of forming a polymer layer using a polymer solution containing the conductive polymer and, if necessary, the conductive material.

In the step, the specific description of the conductive polymer used is as described above, and the coating solution may be prepared by dissolving the polymer in an appropriate solvent. At this time, the type of the solvent is not particularly limited if it can dissolve at least a portion of the conductive polymer.

In the step, the lower limit of the concentration of the conductive polymer present in the polymer solution may be 0.5 wt%, 1 wt%, 1.5 wt%, 2 wt%, 2.5 wt%, or 3 wt% or so, and the upper limit thereof may be 20 wt%, 18 wt%, 16 wt%, 14 wt%, 12 wt%, 10 wt%, 9 wt%, 8 wt%, 7 wt%, 6 wt%, 5 wt%, 4 wt%, or 3 wt% or so. The ratio may be within a range of more than or equal to, or more than any lower limit arbitrarily selected from the above-listed lower limits; or within a range of less than or equal to, or less than any upper limit arbitrarily selected from the above-listed upper limits; or within a range of more than or equal to, or more than any lower limit arbitrarily selected from the above-listed lower limits while being less than or equal to, or less than any upper limit arbitrarily selected from the above-listed upper limits. Such a concentration may be changed as needed.

The conductive polymer may be formed by a known polymerization method. For example, typically, as a method for producing polythiophene, a method using an oxidation polymerization reaction or a method using a radical reaction is known.

A polymer layer is formed on a current collector using the polymer solution. This process typically comprises a step of coating the polymer solution on a current collector and a step of heat-treating the coated coating solution. In this specification, the polymer layer formed on the current collector before the heat treatment may be called a precursor. The crystallinity of the conductive polymer and the orientation of the long-chain hydrocarbon functional group may be controlled by the conditions of the heat treatment.

For example, the heat treatment step may be performed in two steps. For example, the heat treatment step may comprise a first step of primarily heat-treating the precursor at a temperature range $T_1$; and a second step of secondarily heat-treating the precursor at a temperature range $T_2$ following the first step.

The conditions of the first and second steps may be adjusted to achieve the desired orientation or alignment of the conductive polymer and the dispersion state of the conductive material.

For example, the temperature range $T_1$ of the first heat treatment may be adjusted within a predetermined range. For example, the lower limit of the temperature range $T_1$ may be 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, or 140°C or so, and the upper limit thereof may be 300°C, 290°C, 280°C, 270°C, 260°C, 250°C, 240°C, 230°C, 220°C, 210°C, 200°C, 190°C, 180°C, 170°C, 160°C, 150°C, or 140°C or so. The temperature range $T_1$ may be within a range of more than or equal to, or more than any lower limit arbitrarily selected from the above-listed lower limits; or within a range of less than or equal to, or less than any upper limit arbitrarily selected from the above-listed upper limits while being more than or equal to, or more than any lower limit arbitrarily selected from the above-listed lower limits.

In the heat treatment step, the temperature range $T_1$ of the first heat treatment and the temperature range $T_2$ of the second heat treatment may be adjusted. For example, the lower limit of the ratio of the temperature ranges $T_1$ and $T_2$ may be 0.1, 0.2, 0.3, 0.4, 0.5, 0.6, 0.7, 0.8, 0.9, 1, 1.1, or 1.2 or so, and the upper limit thereof may be 5, 4.5, 4, 3.5, 3, 2.5, 2, 1.9, 1.8, 1.7, 1.6, 1.5, 1.4, 1.3, 1.2, or 1.1 or so. The ratio may be within a range of more than, or equal to or more than any lower limit arbitrarily selected from the above-listed lower limits; or within a range of less than or equal to, or less than any upper limit arbitrarily selected from the above-listed upper limits; or within a range of less than or equal to, or less than any upper limit arbitrarily selected from the above-listed upper limits while being more than or equal to, or more than any lower limit arbitrarily selected from the above-listed lower limits.

In one example, in the heat treatment step, the temperature range $T_1$ of the first heat treatment may be adjusted to be higher than the temperature range $T_2$ of the second heat treatment.

The ratio of a heat treatment time $M_1$ in the first heat treatment and a heat treatment time $M_2$ in the second heat treatment may be additionally adjusted. For example, the lower limit of the ratio $M_2/M_1$ may be 1, 2, 3, 4, 5, 6, 7, 8, 9, 10, 11, 12, 13, 14, or 15 or so, and the upper limit thereof may be 1,000, 950, 900, 850, 800, 750, 700, 650, 600, 550, 500, 450, 400, 350, 300, 290, 280, 270, 200, 150, 100, 90, 80, 70, 60, 50, 40, 30, or 20 or so. The ratio $M_2/M_1$ may be within a range of more than or equal to, or more than any lower limit arbitrarily selected from the above-listed lower limits; or within a range of less than or equal to, or less than any upper limit arbitrarily selected from the above-listed upper limits; or within a range of less than or equal to, or less than any upper limit arbitrarily selected from the above-listed upper limits while being more than or equal to, or more than any lower limit arbitrarily selected from the above-listed lower limits.

Here, the lower limit of the secondary heat treatment time $M_2$ may be 0.1 hours, 0.2 hours, 0.3 hours, 0.4 hours, 0.5 hours, 0.6 hours, 0.7 hours, 0.8 hours, 0.9 hours, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, or 18 hours or so, and the upper limit thereof may be 50 hours, 48 hours, 46 hours, 44 hours, 42 hours, 40 hours, 38 hours, 36 hours, 34 hours, 32 hours, 30 hours, 28 hours, 26 hours, 24 hours, 22 hours, 20 hours, 18 hours, 15 hours, 10 hours, 9 hours, 8 hours, 7 hours, 6 hours, 5 hours, 4 hours, 3 hours, 2 hours, or 1.5 hours or so. The second heat treatment time $M_2$ may be within a range of more than or equal to, or more than any lower limit arbitrarily selected from the above-listed lower limits; or within a range of less than or equal to, or less than any upper limit arbitrarily selected from the above-listed upper limits; or within a range of less than or equal to, or less than any upper limit arbitrarily selected from the above-listed upper limits while being more than or equal to, or more than any lower limit arbitrarily selected from the above-listed lower limits.

By the heat treatment as above, the alignment state of the hydrocarbon functional groups, and the like of the conductive polymer is appropriately adjusted, and accordingly, the desired effect can be obtained.

The method of coating the coating solution is not particularly limited, and a known coating method may be applied.

Through the processes, the desired polymer layer and the current collector comprising the same are manufactured. The processes may comprise an appropriate post-treatment process, if necessary.

The manufacturing method may comprise a step of forming the active material layer on the polymer layer on the current collector. The method of forming the active material layer on the polymer layer is not particularly limited as well. Typically, the active material layer is formed by coating a slurry containing the electrode active material, a binder, and a conductive material, and the like on the current collector (on the polymer layer), drying, and then rolling, and such a known method may be applied in the same manner.

The present specification also discloses an electrode assembly or an electrochemical element, for example, a secondary battery, which comprises such an electrode.

The electrochemical element may comprise the electrode as a positive electrode and/or a negative electrode. If the electrode is used as a negative electrode and/or a positive electrode, other constitutions or manufacturing methods of the electrochemical element are not particularly limited, and known methods may be applied.

## Advantageous Effects

The present specification discloses an electrode and a use thereof. The electrode comprises a polymer layer exhibiting a so-called PTC (Positive Temperature Coefficient) effect at a required level at a required time, whereby it can be suitably applied in applications where stability problems due to abnormally high heat or flame are caused. The polymer layer can exhibit oxidation potential characteristics suitable for an electrode use, where such oxidation potential characteristics can be stably maintained even under harsh environments. In the polymer layer of the electrode, the PTC effect can rapidly appear at a required time. The present specification also discloses a use of the electrode.

## Description of Drawings

Figure 1 is a cross-sectional diagram of an exemplary electrode.
Figure 2 is an NMR analysis result for a monomer of Preparation Example 1.

## Mode for Invention

Hereinafter, the electrode, and the like are specifically described through examples and comparative examples, but the electrode and the like are not limited to the following examples.

### 1. NMR analysis

$^{1}H$-NMR analyses were performed at room temperature using an NMR spectrometer including a Bruker UltraShield spectrometer (300 MHz) with a 5 mm triple resonance probe. A sample was diluted in a solvent ($CDCl_3$) for NMR measurement to a concentration of about 10 mg/ml or so and used, and the chemical shift was expressed in ppm.

### 2. GPC (Gel Permeation Chromatograph)

Molecular weight characteristics were measured using GPC (Gel permeation chromatography). A sample is placed in a 5 mL vial and diluted with chloroform to a concentration of about 1 mg/mL or so. The standard sample for calibration and the sample to be analyzed were filtered through a syringe filter (pore size: 0.45 μm), and then the molecular weight characteristics were measured. As the analysis program, Waters' Empower 3 was used, and a weight average molecular weight (Mw) and a number average molecular weight (Mn) were each obtained by comparing the elution time of the sample with the calibration curve, and the molecular weight distribution (PDI) was calculated by the ratio (Mw/Mn).
The measurement conditions of GPC are as follows.

<GPC measurement conditions>

Device: Waters' 2414
Column: using 3 Styragels from Waters
Solvent: THF (tetrahydrofuran)
Column temperature: 35°C
Sample concentration: 1mg/mL, 1μL injection
Standard sample: polystyrene (Mp: 3900000, 723000, 316500, 52200, 31400, 7200, 3940, 485)

### 3. Thickness measurement

The thickness of the polymer layer, and the like was measured by cross-sectioning it using an ion milling device (Hitachi, IM5000), and then photographing an image with a SEM (Scanning Electron Microscope) (JEOL, JSM-7200F). The conditions for forming the cross section by the ion milling were performed by setting the equipment in cross-section milling mode, the speed (reciprocation/min) of 3, the acceleration voltage of 6.0kV, the discharge voltage of 15 kV, the current of 150 μA, and the time of 4 hours.

### 4. Measurement of oxidation potential (polymer layer)

An oxidation potential was measured in the following manner. A polymer layer was formed to a thickness of about 10 μm or so on an aluminum foil (Al Foil) having a thickness of about 15 μm using a conductive copolymer. The polymer layer was formed in the same manner as described in each example or comparative example, and was formed to a thickness of about 10 μm or so. A separator and a lithium film were laminated on the polymer layer to manufacture a laminate in which the aluminum foil/polymer layer/separator/lithium film were laminated, and the laminate was punched into a circle with a diameter of about 1.4 cm. A coin cell was manufactured using the circularly punched laminate and an electrolyte (using the Welcos CR2032 coin cell kit). As the separator, the WL20C model from W Scope Korea was used; as the lithium film, a lithium film with a thickness of about 100 μm or so was used; and as the electrolyte, Enchem's product (1M $LiPF_6$ solution (solvent: EC/DMC/EMC =3/4/3 (mass ratio), EC: Ethylene Carbonate, DMC: dimethyl carbonate, EMC: ethylmethyl carbonate) was used.
The oxidation potential of the coin cell was measured using an electrochemical meter (potentiostat) (Princeton Applied Research, PARASTAT-MC) at 25°C. The oxidation potential was measured by measuring CV (Cyclic Voltammetry) in the range of 1.5V to 5.5V at a scan rate of 0.17 mV/sec to 0.5 mV/sec.

### 5. Measurement of oxidation potential (electrode active material)

With respect to an oxidation potential of an electrode active material, a coin cell was manufactured using an electrode manufactured using the electrode active material, and it was measured for the relevant coin cell.
A slurry was prepared by formulating an electrode active material to be measured, a conductive material (ECP (Ketjen Black) 0.5%, SFG (Trimrex graphite) 0.4%, DB (Denka Black) 0.4%), PVDF (poly(vinylidene fluoride)), and NMP (N-Methyl-2-pyrrolidone) in a weight ratio of 75:1:1:23 (electrode active material: conductive material: PVDF: NMP). The

slurry was applied onto the current collector with a doctor blade, dried at room temperature (about 25°C), and then maintained in a drying oven at 130°C for 30 minutes or so, and then rolled to form an electrode active material layer with a thickness of about 53 μm or so, whereby an electrode was manufactured. The rolling was performed so that the active material layer had a void ratio of about 25% or so. The void ratio of the active material layer is a value calculated by a method of comparing the ratio of the difference between the actual density and the density after rolling, and the method of obtaining the void ratio in such a manner is known. An aluminum foil with a thickness of about 15μm or so was used as the current collector.

A separator and a lithium film were laminated on the electrode active material layer of the electrode to manufacture a laminate in which the electrode/separator/lithium film were laminated, and the laminate was punched into a circle with a diameter of about 1.4 cm. A coin cell was manufactured using the circularly punched laminate and an electrolyte (using the Welcos CR2032 coin cell kit).

As the separator, the WL20C model from W Scope Korea was used; as the lithium film, a film with a thickness of about 100 μm or so was used; and as the electrolyte, 1M $LiPF_6$ solution (solvent: EC/DMC/EMC =3/4/3 (mass ratio), EC: Ethylene Carbonate, DMC: dimethyl carbonate, EMC: ethylmethyl carbonate)), which was Enchem's product, was used.

The oxidation potential of the coin cell was measured using an electrochemical meter (potentiostat) (Princeton Applied Research, PARASTAT-MC) at 25°C. The oxidation potential was measured by measuring CV (Cyclic Voltammetry) in the range of 1.5V to 5.5V at a scan rate of 0.17 mV/sec to 0.5 mV/sec.

The oxidation potential was measured based on $Li/Li^+$.

Figure 2 shows results of measuring an oxidation potential by applying NCM ($Li[Ni_xCo_{(1-x)/2}Mn_{(1-x)/2}]O_2$, x = 0.8) as an electrode active material. The oxidation potential of the electrode active material was about 3.7 V.

**6. DC resistance measurement method**

DC resistance was evaluated using the same coin cell used in the oxidation potential measurement. A voltage of 4.3eV was applied to the coin cell for 10 minutes at room temperature (25°C), and the DC resistance was measured using a Fluke digital multi-tester (FLUKE-87-5).

**7. AC impedance resistance**

AC impedance resistance was evaluated through EIS (Electrochemical Impedance Spectronization) using the same coin cell used in the oxidation potential measurement. A voltage of 4.3V was applied to the coin cell for 10 minutes at room temperature (25°C), and a Nyquist plot was obtained by an EIS measurement method at 50,000 Hz to 0.1 Hz, and the AC impedance resistance obtained in the high frequency region was measured. An electrochemical meter (potentiostat) (Princeton Applied Research, PARASTAT-MC) was used as the EIS measurement device.

**8. Measurement of maximum resistance change rate (DC resistance)**

A maximum resistance change rate ΔR1 is determined according to Equation 2 below.

<Equation 2>

$$\Delta R1 = \mathrm{Max}\{(R_{n+5}/R_n)/5\}$$

In Equation 2, $R_n$ is the DC resistance at an arbitrary temperature n°C within the range of 25°C to 135°C, and $R_{n+5}$ is the DC resistance at a temperature ((n+5)°C) that is 5°C higher than the temperature n°C.

The above ΔR1 is measured in the following manner.

A coin cell for DC resistance measurement is placed in the center of a convection oven (Jeotech, OF3-05W), and the temperature of the oven is set so that an initial temperature is 25°C, a final temperature is 135°C, and the temperature increases by 5°C per minute. The coin cell is connected to a resistance measurement multimeter (Fluke's digital multi-tester (FLUKE-87-5)) outside the oven to enable the resistance measurement. Subsequently, the DC resistance is measured at each temperature (measurement up to 135°C while increasing the measurement temperature by 5°C in the order of 25°C, 30°C, 35°C, and 40°C) in a state where the temperature increases, as set. $R_n$ and $R_{n+5}$ of Equation 1 for each measurement temperature are each measured, $R_{n+5}/R_n$ ($R_{30}/R_{25}$, $R_{35}/R_{30}$ ~ $R_{135}/R_{130}$) are calculated, and then they are divided by 5 again.

After obtaining the above $(R_{n+5}/R_n)/5$ in the temperature range of 25°C to 135°C, the maximum value among them is obtained as the above ΔR1. Through the above ΔR1, the temperature reactivity of the resistance increase of the conductive copolymer (polymer layer) at an on-set temperature can be confirmed.

The on-set temperature is the temperature n°C at which $(R_{n+5}/R_n)/5$ shows the maximum value.

As the coin cell for the DC resistance measurement, the same one applied upon measuring the oxidation potential was used.

### 9. Measurement of maximum resistance change rate (AC impedance)

The maximum resistance change rate ΔR2 is determined according to Equation 3 below.

<Equation 3>

$$\Delta R2 = \mathrm{Max}\{(R_{z+5}/R_z)/5\}$$

In Equation 3, $R_z$ is the AC impedance resistance at an arbitrary temperature z°C within the range of 25°C to 135°C, and $R_{z+5}$ is the AC impedance resistance at a temperature ((z+5)°C) that is 5°C higher than the temperature z°C.

The above ΔR2 is measured in the following manner.

A coin cell for AC impedance resistance measurement is placed in the center of the convection oven (Jeotech, OF3-05W), and the temperature of the oven is set so that an initial temperature is 25°C, a final temperature is 135°C, and the temperature increases by 5°C per minute. The coin cell is connected to a resistance measuring device (potentiostat) (Princeton Applied Research, PARASTAT-MC) outside the oven to enable resistance measurement. Subsequently, the AC impedance resistance is measured at each temperature (measurement up to 135°C while increasing the measurement temperature by 5°C in the order of 25°C, 30°C, 35°C, and 40°C) in a state where the temperature increases, as set. $R_z$ and $R_{z+5}$ of Equation 2 for each measurement temperature are each measured, and $R_{z+5}/R_z$ ($R_{30}/R_{25}$, $R_{35}/R_{30}$ ~ $R_{135}/R_{130}$) are calculated and then they are divided by 5 again.

After obtaining the above $(R_{z+5}/R_z)/5$ in the temperature range of 25°C to 135°C, the maximum value among them is obtained as the above ΔR2.

Through the above ΔR2, the temperature reactivity of the resistance increase of the conductive copolymer (polymer layer) at an on-set temperature can be confirmed.

The on-set temperature is the temperature n°C at which $(R_{z+5}/R_z)/5$ shows the maximum value.

As the coin cell for the measurement, the same one applied upon measuring the oxidation potential was used.

The AC impedance resistance was determined as the resistance obtained from the semicircle in the high frequency region by applying a voltage of 4.3V for 10 minutes, and obtaining a Nyquist plot using the EIS measurement method at 50,000 Hz to 0.1 Hz.

### 10. Charge and discharge test

A charge-and-discharge test was performed by manufacturing a coin cell. The coin cell was manufactured using a CR2032 standard coin cell kit (Welkos CR2032 coin cell kit). The electrode prepared in each of Examples or Comparative Examples was used as a positive electrode, and a lithium film (thickness: about 100 μm) was used as a negative electrode. As an electrolyte, a 1M $LiPF_6$ solution (solvent: EC/DMC/EMC=3/4/3 (mass ratio), EC: ethylene carbonate, DMC: dimethyl carbonate, EMC: ethylmethyl carbonate), which was Enchem's product, was used, and as a separator, a PE (poly(ethylene)) separator (W-Scope Korea, WL20C model) was used.

A charge-and-discharge test was performed using the coin cell, and the test was performed using the constant current-constant voltage (CC-CV) method, which was performed using the charge/discharge conditions as 0.1C/0.1C (condition 1), 0.5C/0.1C (condition 2), 0.5C/0.5C (condition 3), 0.5C/1C (condition 4), and 0.5C/2C (condition 5), respectively.

### 11. High-temperature discharge capacity reduction rate

A coin cell was manufactured in the same manner as in the charge/discharge test.

The coin cell was charged/discharged once at 25°C, and the capacity at 0.2C was set as the room temperature discharge capacity ($C_{25}$). The one charge/discharge means that a process of charging it in a CC (constant current)/CV (constant voltage) method at a rate of 0.2C by setting a final charging voltage of 4.5V and a final charging current of 1mA, and discharging it in a CC (constant current) method at a rate of 0.2C by setting a final discharging voltage of 3.0V is repeated once as one cycle.

Meanwhile, after maintaining the coin cell at 130 for about 10 minutes, the high-temperature discharge capacity ($C_{130}$) was obtained by the following method. For the coin cell maintained at the high temperature, as a process of charging it in a CC (constant current)/CV (constant voltage) method at a rate of 0.5C by setting a final charging voltage of 4.5V and a

final charging current of 1mA, and discharging it in a CC (constant current) method at a rate of 2C by setting a final discharging voltage of 3.0V again was set to one cycle, the cycle was repeated 30 times, and the discharge capacity after 30 charge/discharge cycles was applied as the high-temperature discharge capacity ($C_{130}$). The 30 charge/discharge cycles were performed at 45°C.

**12. Average particle diameter**

An average particle diameter (D50 particle diameter) of conductive particles was measured using a MASTERSIZER3000 device from Marvern in accordance with ISO-13320 standard. Toluene was used as a solvent upon measurement. If a sample (conductive particles) is dispersed in the solvent and the laser is irradiated, the laser is scattered by the sample dispersed in the solvent. Since the intensity and directionality of the scattered laser vary depending on the size of the particle, the average particle diameter can be obtained by analyzing this using a Mie theory. Through the above analysis, the measurement results were converted into particle diameters of spheres having the same volume as the dispersed sample to obtain a volume-based cumulative graph of particle size distribution, and the particle diameter (median particle diameter) at 50% accumulation of the graph was designated as the average particle diameter (D50 particle diameter).

**Preparation Example 1. Synthesis of monomer (A)**

A monomer of Formula A below was synthesized in the following manner.

[Formula A]

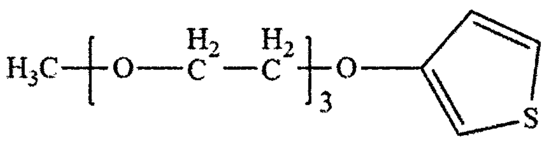

3 g (26.28 mmol, 1 eq) of 3-methoxythiophene and 7.03 g (39.42 mmol, 1.5 eq) of triethylene glycol monomethyl ether were dissolved together with 500 mg of p-toluenesulfonic acid (p-TsOH) (2.63 mmol, 0.1 eq) in 150 ml of toluene, and mixed. The mixture was refluxed at 120°C under a nitrogen atmosphere and simultaneously reacted, whereby methanol produced by the reaction (transetherification) was removed by 4A type molecular sieves filled in an extractor (soxhlet extractor). The reactant was cooled to room temperature after reflux for 24 hours, and then quenched with water, and extracted with ethyl acetate, and then washed with brine, and dried over magnesium sulfate ($MgSO_4$). The solvent was removed using a rotary evaporator, and the residue was purified by column chromatography eluting with methylene chloride/hexane (2:1) to obtain the target compound (monomer (A)). The NMR analysis results for the monomer (A) are as shown in Figure 2.

**Preparation Example 2. Synthesis of conductive polymer (A)**

To a solution in which 3.20 g (19.71 mmol, 3 eq) of iron (III) chloride was dissolved in 150 ml of methylene chloride. 0.7 g (2.96 mmol, 0.6 eq) of 3-dodecylthiophene, 0.3 g (1.48 mmol, 0.3 eq) of 3-hexylthiophene, 0.12 g (0.49 mmol, 0.1 eq) of the monomer (A) of Preparation Example 1, and 0.37 g (1.23 mmol, 0.3 eq) of pyrrole were introduced, and polymerized at 25°C for 24 hours to prepare a conductive polymer (A). In the conductive polymer (A), the molar ratio of the 3-dodecylthiophene unit (I), the 3-hexylthiophene unit (II), the monomer (A) unit (III) of Preparation Example 2, and the pyrrole (IV) unit was 2.96:1.48:0.49:1.23 (I: II: III: IV) or so. The polymerization solution was placed in an osmosis membrane with an MWCO (molecular weight of cut-off) of 5000, and then immersed in 200 ml of an acetonitrile solvent to remove unreacted iron chloride and monomers. The residue precipitated inside the osmosis membrane was washed with methanol, and dried at 60°C for 12 hours to obtain a conductive polymer (A). The weight average molecular weight (Mw) and number average molecular weight (Mn) of the conductive polymer (A) were 99,000 g/mol and 28,000 g/mol or so, respectively. The oxidation potential of the conductive polymer was about 3.95 V or so (based on $Li/Li^+$).

**Preparation Example 3. Polydopamine-coated conductive particles**

Carbon black particles (IMERYS, C-NERGY™ SUPER C65) were used as the conductive particles. The average particle diameter (D50 particle diameter) of the conductive particles was about 60 nm or so. DHC (dopamine hydrochloride) (CAS No. 62-31-7) was added to a buffer solution and stirred at room temperature (about 25°C). As the buffer solution, BIOSESANG's 0.1M pH 8.5 Tris-buffer product was used. The molar concentration of DHC in the final solution

was about 2 mg/mL or so. In a mixture of the buffer solution and DHC, the conductive particles were dispersed (sonication for 1 hour) at a concentration of about 4 mg/mL, and stirred additionally for about 18 hours to form a coating layer of polydopamine on the conductive particles. After filtering under reduced pressure using a paper filter, the resultant particles were vacuum-dried to obtain conductive particles coated with polydopamine.

**Example 1.**

An Al foil having a thickness of about 15 μm or so was used as a current collector.

The conductive polymer (A) of Preparation Example 2 and the conductive particles (P) of Preparation Example 3 were mixed in a weight ratio (A: P) of 8:2, and the mixture was dispersed in a solvent (chloroform) at a concentration of 2 wt% or so to prepare a coating liquid. The coating liquid was coated on the current collector in a bar coating manner, and heat-treated (drying) at 140°C for 4 minutes or so, and then heat-treated (annealing) again at 130°C for 1 hour or so to form a polymer layer having a thickness of about 300 nm.

The oxidation potential of the polymer layer was about 3.45 V or so when measured using the above-described method.

An active material layer was formed on the polymer layer. The active material layer was formed by applying a slurry containing an electrode active material, a carbon-based conductive material (ECP (Ketjen Black) 0.5%, SFG (Trimrex graphite) 0.4%, DB (Denka Black) 0.4%), PVDF (polyvinylidene fluoride), and NMP (N-Methyl-2-pyrrolidone) in a weight ratio of 75:1:1:23 (electrode active material: conductive material: PVDF: NMP) to a thickness of about 90 μm or so on the polymer layer using a doctor blade, drying at room temperature (about 25°C), and then further drying under vacuum conditions at 120°C. Subsequently, rolling was performed to have a void ratio of about 25% or so, thereby manufacturing an electrode. As the electrode active material, NCM ($Li[Ni_xCo_{(1-x)/2}Mn_{(1-x)/2}]O_2$, x = 0.8) (oxidation potential: about 3.7 V) was used.

**Example 2.**

A polymer layer and an electrode were manufactured in the same manner as in Example 1, except that the coating liquid was prepared by mixing the conductive polymer (A) of Preparation Example 2 and the conductive particles (P) of Preparation Example 3 in a weight ratio of 6:4 (A: P). The oxidation potential of the polymer layer was about 3.41 V or so when measured by the above-described method.

**Example 3.**

A polymer layer and an electrode were manufactured in the same manner as in Example 1, except that the coating liquid was prepared by mixing the conductive polymer (A) of Preparation Example 2 and the conductive particles (P) of Preparation Example 3 in a weight ratio of 5:5 (A: P). The oxidation potential of the polymer layer was about 3.37 V or so when measured by the above-described method.

**Comparative Example 1.**

An electrode was manufactured in the same manner as in Example 1, except that the polymer layer was not formed.

**Comparative Example 2.**

A polymer layer and an electrode were manufactured in the same manner as in Example 1, except that the coating liquid was prepared using only the conductive polymer (A) of Preparation Example 2, without using the conductive particles of Preparation Example 3. The oxidation potential of the polymer layer was about 3.72 V or so when measured by the above-described method.

For the manufactured electrodes, the oxidation potential (Vs) (based on $Li/Li^+$) of the polymer layer, the oxidation potential (Va) (based on $Li/Li^+$) of the electrode active material, ΔV, DC resistance, AC impedance, and the PTC effect were measured, and the results were summarized in Table 1 below. The ΔV was calculated by the equation $100 \times (Va - Vs)/Va$ (Va: oxidation potential of the electrode active material, Vs: oxidation potential of the polymer layer). In Table 1, the unit of oxidation potential is V.

[Table 1]

| | | Example | | | Comparative Example | |
|---|---|---|---|---|---|---|
| | | 1 | 2 | 3 | 1 | 2 |
| Vs | | 3.45 | 3.41 | 3.37 | - | 3.6 |
| Va | | 3.7 | 3.7 | 3.7 | 3.7 | 3.7 |
| ΔV | | 6.8 | 7.8 | 8.9 | - | 2.7 |
| DC resistance (Ω·cm) | | 75 | 61 | 52 | 4.7 | 370 |
| AC impedance (Ω) | | 10 | 7.2 | 6.2 | 1.7 | 42 |
| Equation 1 | ΔR1 | 195 | 182 | 165 | 2.1 | 210 |
| | On set (°C) | 95 | 100 | 95 | - | 95 |
| Equation 2 | ΔR2 | 19 | 18.1 | 17.4 | 2.8 | 22.7 |
| | On set (°C) | 95 | 100 | 95 | - | 95 |

In Table 2 below, the capacities measured according to the charge/discharge test are summarized for each condition.

[Table 2]

| | Example | | | Comparative Example | |
|---|---|---|---|---|---|
| | 1 | 2 | 3 | 1 | 2 |
| Condition 1 (0.1C/0.1C) | 219 | 219.5 | 220.1 | 220.3 | 217.7 |
| Condition 2 (0.5C/0.1C) | 218.8 | 219.6 | 219.8 | 220.1 | 217.7 |
| Condition 3 (0.5C/0.5C) | 197.0 | 197.5 | 197.8 | 198.0 | 194.3 |
| Condition 4 (0.5C/1C) | 192.9 | 193.6 | 193.8 | 194.0 | 190.7 |
| Condition 5 (0.5C/2C) | 186.4 | 187.0 | 187.4 | 187.4 | 179.2 |

From the results of Table 2, in the case of Examples, the characteristics are equivalent to those of Comparative Example 1, to which the polymer layer is not applied, but in the case of Comparative Example 2, where the conductive particles are not applied and the oxidation potential is not adjusted in relation to the electrode active material, it can be confirmed that a potential drop occurs during discharge due to an increase in the C-rate.

In Table 3 below, the room temperature discharge capacity $C_{25}$ and the high temperature discharge capacity $C_{130}$ according to the high temperature discharge capacity reduction rate measurement method are summarized. In Table 3, the reduction rate was calculated as $100 \times (C_{25}-C_{130})/C_{25}$.

[Table 3]

| | Example | | | Comparative Example | |
|---|---|---|---|---|---|
| | 1 | 2 | 3 | 1 | 2 |
| C25 | 219.5 | 220.0 | 220.5 | 221.3 | 218.0 |
| C130 | 22.5 | 39.0 | 46.5 | 168.5 | 21.8 |
| Reduction rate | 89.7 | 82.3 | 78.9 | 23.9 | 90.0 |

Through Table 3, it can be confirmed that when the electrode according to the present application is applied, the decrease rate of the discharge capacity is high under high temperature conditions, and accordingly, stability can be secured in an abnormal state.

In the case of Comparative Example 2, a decrease in the discharge capacity was confirmed under high temperature conditions, but the room temperature discharge capacity under the same conditions was lower than those of Examples.

## Claims

**1.** An electrode comprising:

a current collector;
an active material layer formed on the current collector and including an electrode active material; and
a polymer layer existing between the current collector and the active material layer, and including a conductive polymer and a conductive material, wherein
an oxidation potential of the polymer layer based on $Li/Li^+$ is lower than an oxidation potential of the electrode active material based on $Li/Li^+$.

**2.** The electrode according to claim 1, wherein ΔV by Equation 1 below is more than 5% and 20% or less:

[Equation 1]

$$\Delta V = 100 \times (Va - Vs)/Va$$

wherein, Va is the oxidation potential of the electrode active material based on $Li/Li^+$, and Vs is the oxidation potential of the polymer layer based on $Li/Li^+$.

**3.** The electrode according to claim 1, wherein the oxidation potential of the polymer layer based on $Li/Li^+$ is 5.0 V or less.

**4.** The electrode according to claim 1, wherein an absolute value of ΔR4 of Equation 4 below is 50% or more:

[Equation 5]

$$\Delta R4 = 100 \times (C_1 - C_3)/C_1$$

wherein, $C_1$ is a discharge capacity at 25°C, and $C_3$ is a discharge capacity after storage at 130°C for 10 minutes.

**5.** The electrode according to claim 1, wherein the conductive polymer has a long-chain hydrocarbon functional group.

**6.** The electrode according to claim 5, wherein the conductive polymer contains, as the long-chain hydrocarbon functional group, a first hydrocarbon functional group with 10 or more carbon atoms and a second hydrocarbon functional group with 9 or less carbon atoms.

**7.** The electrode according to claim 1, wherein the conductive polymer contains a functional group of Formula 1 below:

[Formula 1]

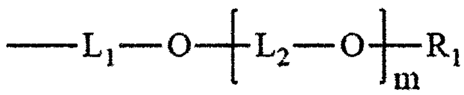

wherein, $L_1$ is a single bond, an alkylene group or an alkylidene group, $L_2$ is an alkylene group or an alkylidene group, $R_1$ is hydrogen or an alkyl group, and m is a number in a range of 1 to 10.

**8.** The electrode according to claim 1, wherein the conductive polymer contains a thiophene monomer unit and a no-thiophene monomer unit.

**9.** The electrode according to claim 8, wherein the no-thiophene monomer unit is an aromatic monomer unit or a nitrogen-containing heterocyclic monomer unit.

**10.** The electrode according to claim 8, wherein the molar ratio of the thiophene monomer unit in the conductive polymer is 70 mol% or more, and 0.05 mol or more of the no-thiophene monomer unit exists per 1 mol of the thiophene monomer unit.

**11.** The electrode according to claim 8, the no-thiophene monomer unit is at least one unit selected from the group consisting of units of Formulas 10 to 15 below:

[Formula 10]

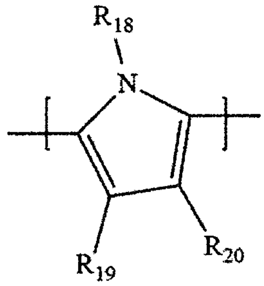

wherein, $R_{18}$, $R_{19}$, and $R_{20}$ are each independently hydrogen, a polar functional group, or a hydrocarbon functional group:

[Formula 11]

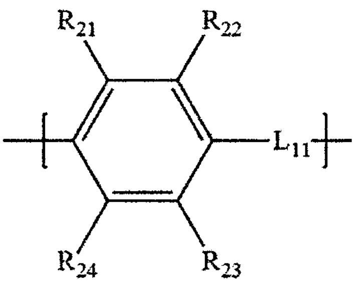

wherein, $L_{11}$ is a single bond, an alkylene group, an alkylidene group, O, or $NR^1$, where $R^1$ is hydrogen, an alkyl group, an alkenyl group, or an aryl group:

[Formula 12]

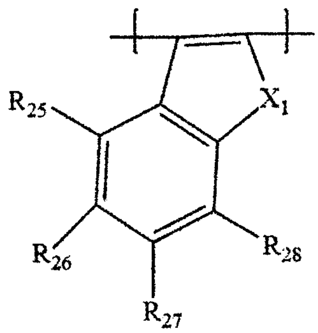

wherein, $X_1$ is S, O, or $NR^1$, where $R^1$ is hydrogen, an alkyl group, an alkenyl group, or an aryl group, and $R_{25}$ to $R_{28}$ are each independently hydrogen, an alkyl group, an alkenyl group, an alkoxy group, an aryl group, or halogen:

[Formula 13]

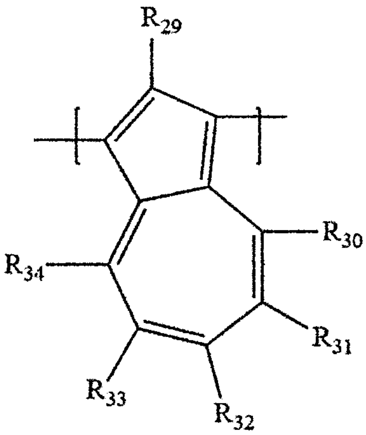

wherein, $R_{29}$ to $R_{34}$ are each independently hydrogen, an alkyl group, an alkenyl group, an alkoxy group, an aryl group, a hydroxy group, or halogen:

[Formula 14]

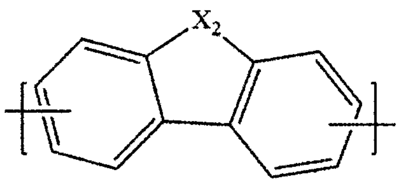

wherein, $X_2$ is $CR^aR^b$, or $NR^1$, where $R^a$, $R^b$ and $R^l$ are each independently hydrogen, an alkyl group, an alkenyl group, or an aryl group, and the unit of Formula 14 above is unsubstituted or substituted with one or more selected from the group consisting of an alkyl group, an alkenyl group, an alkoxy group, an aryl group, and halogen:

[Formula 15]

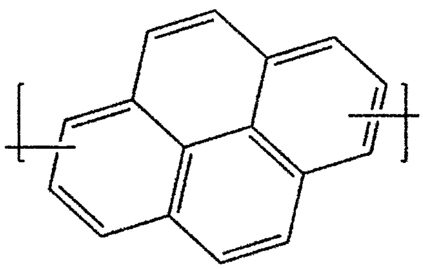

wherein, the unit of Formula 15 is unsubstituted or substituted with one or more selected from the group consisting of an alkyl group, an alkenyl group, an alkoxy group, an aryl group, and halogen.

**12.** The electrode according to claim 1, wherein the conductive material is carbon particles, carbon fibers, graphene, graphite, carbon black, or carbon nanotubes.

**13.** The electrode according to claim 1, wherein the conductive material is surface-treated with a polyphenol-based compound.

**14.** An electrode assembly comprising the electrode of any one of claims 1 to 13.

**15.** A secondary battery comprising the electrode assembly of claim 14.

[Figure 1]

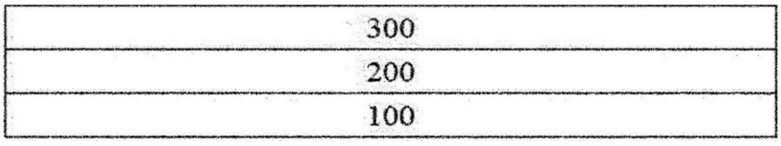


[Figure 2]

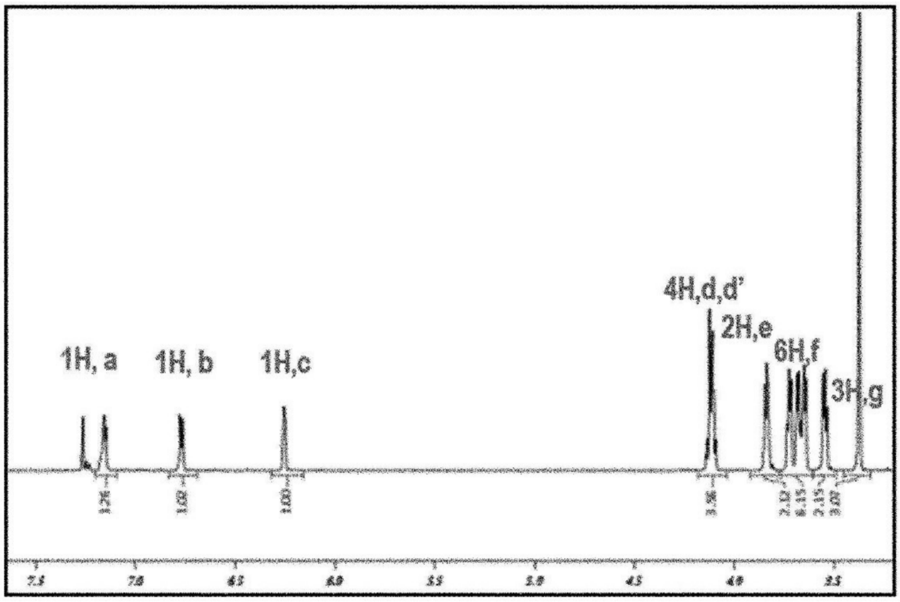

## INTERNATIONAL SEARCH REPORT

International application No.
**PCT/KR2024/012240**

**A. CLASSIFICATION OF SUBJECT MATTER**

**H01M 4/13**(2010.01)i; **H01M 4/62**(2006.01)i; **H01M 10/42**(2006.01)i; **H01M 10/052**(2010.01)i; **H01M 50/586**(2021.01)i; **H01M 4/02**(2006.01)i

According to International Patent Classification (IPC) or to both national classification and IPC

**B. FIELDS SEARCHED**

Minimum documentation searched (classification system followed by classification symbols)

H01M 4/13(2010.01); H01M 10/052(2010.01); H01M 4/62(2006.01); H01M 50/403(2021.01); H01M 50/417(2021.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CAplus) & keywords: 고분자(polymer), 티오펜(thiophene), 산화 전위(oxidation potential), 전극(electrode), 집전체(current collector), 활물질(active material)

**C. DOCUMENTS CONSIDERED TO BE RELEVANT**

| Category* | Citation of document, with indication, where appropriate, of the relevant passages | Relevant to claim No. |
|---|---|---|
| X<br>Y | LI, H. et al. Building a thermal shutdown cathode for Li-Ion batteries using temperature-responsive poly (3-dodecylthiophene). Energy technology. 2020, vol. 8, article no. 2000365, pp. 1-9.<br>See abstract; pages 1-3 and 8; and figures 1(b), 2(a), 2(b), 2(c) and 6. | 1-5,12,14,15<br>6-11,13 |
| Y | KR 10-2023-0033788 A (LG ENERGY SOLUTION, LTD.) 09 March 2023 (2023-03-09)<br>See claims 1 and 3; and paragraph [0049]. | 6,8-11 |
| Y | JP 2010-135310 A (SANYO CHEMICAL IND. LTD.) 17 June 2010 (2010-06-17)<br>See claims 1 and 3; and paragraphs [0051] and [0052]. | 7 |
| Y | KR 10-2015-0027115 A (TORAY INDUSTRIES, INC.) 11 March 2015 (2015-03-11)<br>See abstract; claims 1-3; and paragraphs [0012]-[0014]. | 13 |
| A | JP 2013-041785 A (TORAY IND. INC.) 28 February 2013 (2013-02-28)<br>See claims 1-5; and paragraphs [0033]-[0035]. | 1-15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 December 2024** | **02 December 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**
**Information on patent family members**

International application No.
**PCT/KR2024/012240**

| Patent document cited in search report | Publication date (day/month/year) | Patent family member(s) | Publication date (day/month/year) |
|---|---|---|---|
| KR 10-2023-0033788 A | 09 March 2023 | None | |
| JP 2010-135310 A | 17 June 2010 | None | |
| KR 10-2015-0027115 A | 11 March 2015 | CA 2872445 A1 | 12 December 2013 |
| | | CA 2872445 C | 07 April 2020 |
| | | CN 103466602 A | 25 December 2013 |
| | | CN 104271497 A | 07 January 2015 |
| | | CN 104271497 B | 14 September 2016 |
| | | EP 2858945 A1 | 15 April 2015 |
| | | EP 2858945 B1 | 20 September 2017 |
| | | ES 2642192 T3 | 15 November 2017 |
| | | JP 2015-520109 A | 16 July 2015 |
| | | JP 6098714 B2 | 22 March 2017 |
| | | KR 10-2000812 B1 | 16 July 2019 |
| | | TW 201400408 A | 01 January 2014 |
| | | TW I580637 B | 01 May 2017 |
| | | US 10347916 B2 | 09 July 2019 |
| | | US 2015-0140438 A1 | 21 May 2015 |
| | | WO 2013-181994 A1 | 12 December 2013 |
| JP 2013-041785 A | 28 February 2013 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020230108455

### Non-patent literature cited in the description

- *CHEMICAL ABSTRACTS*, 62-31-7